# EUROPEAN PATENT SPECIFICATION

(11) **EP 3 946 093 B1**
(45) Date of publication and mention of the grant of the patent: **26.04.2023**
(21) Application number: 20720251.6
(22) Date of filing: 25.03.2020
(51) Int. Cl.: A61B 17/32

(54) **ULTRASONIC SURGICAL INSTRUMENTS FOR SEALING, CUTTING, AND/OR SENSING TISSUE**
ULTRASONISCHE CHIRURGISCHE INSTRUMENTE ZUM DICHTEN, SCHNEIDEN UND / ODER SINNEN VON GEWEBE
INSTRUMENTS CHIRURGICAUX ULTRASONIQUES POUR SCELLER, COUPER ET / OU DÉTECTER UN TISSU

(30) Priority: 26.03.2019 US 201962823769 P; 26.03.2019 US 201962823875 P
(43) Date of publication of application: 09.02.2022
(73) Proprietor: Covidien LP, Mansfield, MA 02048 (US)
(72) Inventor: COWLEY, Michael S., Boulder, Colorado 80301 (US); VAN TOL, David J., Boulder, Colorado 80301 (US); LYONS, Michael B., Boulder, California 80301 (US)
(74) Representative: Maschio, Antonio
(86) International application number: PCT/US2020/024732
(87) International publication number: WO 2020/198372

(56) References cited:
- EP-A1- 2 745 792
- US-A1- 2012 136 353
- US-B2- 9 326 787
- US-B2- 9 375 205

## Description

### CROSS-REFERENCE TO RELATED APPLICATIONS

This application claims the benefit of, and priority to, U.S. Provisional Patent Application Nos. 62/823,769 and 62/823,875, both filed on March 26, 2019.

### FIELD

The present disclosure relates to surgical instruments, systems, and exemplary methods. More specifically, the present disclosure relates to ultrasonic and multi-energy surgical instruments, systems, and exemplary methods, not forming part of the invention, for sealing, cutting, and/or sensing tissue.

### BACKGROUND

Ultrasonic surgical instruments and systems utilize ultrasonic energy, i.e., ultrasonic vibrations, to treat tissue. More specifically, ultrasonic surgical instruments and systems utilize mechanical vibration energy transmitted at ultrasonic frequencies to coagulate, cauterize, fuse, seal, cut, and/or desiccate tissue to effect hemostasis.

Ultrasonic surgical instruments typically employ a transducer coupled to a handle of the ultrasonic surgical instrument and configured to produce ultrasonic energy for transmission along a waveguide to an end effector of the ultrasonic surgical instrument that is designed to treat tissue with the ultrasonic energy. The transducer may be driven by an ultrasonic generator that is on-board, e.g., on or within the handle of the ultrasonic surgical instrument, or remotely disposed, e.g., as a set-top box connected to the ultrasonic surgical instrument via a surgical cable. The end effector of the ultrasonic surgical instrument may include a blade that receives the ultrasonic energy from the waveguide for application to tissue and a jaw member configured to clamp tissue between the blade and the jaw member to facilitate treatment thereof. US Patent 9,326,787 discloses an ultrasonic instrument known from the prior art in the field of the application.

### SUMMARY

The main embodiments of the invention are defined by independent claims 1 and 12. preferred embodiments are defined in the dependent claims.

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

Provided in accordance with aspects of the present disclosure is an ultrasonic surgical instrument including a housing, an ultrasonic transducer supported by the housing, and an elongated assembly extending distally from the housing. The elongated assembly includes a waveguide configured to operably couple to the ultrasonic transducer. The waveguide defines a blade at a distal end portion thereof. The elongated assembly further includes first and second panels disposed on opposite sides of the blade in laterally-spaced relation relative thereto and extending along at least a portion of a length of the blade, and jaw pivotable relative to the blade and the first and second panels between a spaced-apart position and an approximated position. In the approximated position, the blade and the jaw are configured to define a first distance therebetween and grasp a center portion of tissue therebetween under a first clamping force, and the jaw and each of the first and second panels are configured to define a second distance therebetween greater than the first distance and grasp outer lateral portions of tissue therebetween under a second clamping pressure less than the first clamping pressure. In other aspects, the second distance may be substantially equal to the first distance or less than the first distance such that the second clamping pressure is substantially equal to or greater than the first clamping pressure.

In an aspect of the present disclosure, the blade includes a tissue-contacting surface having a convex configuration defining an apex. In such aspects, in the clamping position, the tissue-contacting surface of the blade and the jaw are configured to grasp a center portion of tissue therebetween.

In another aspect of the present disclosure, the apex of the tissue-contacting surface of the blade extends further towards the jaw as compared to tissue-contacting surfaces of the first and second panels.

In still another aspect of the present disclosure, the jaw includes a structural body and a jaw liner. The jaw liner defines a tissue-contacting surface and the structural body defines first and second tissue-contacting surfaces disposed on either side of the tissue-contacting surface of the jaw liner. In such aspects, in the approximated position, the tissue-contacting surface of the jaw liner and the blade are configured to grasp a center portion of tissue therebetween, and the first and second tissue-contacting surfaces of the structural body and the first and second panels, respectively, are configured to grasp outer lateral portions of tissue therebetween.

In yet another aspect of the present disclosure, the first and second tissue-contacting surfaces of the structural body are recessed relative to the tissue-contacting surface of the jaw liner. In other aspects, the first and second tissue-contacting surfaces of the structural body are substantially level, e.g., co-planar, relative to the tissue-contacting surface of the jaw liner or protrude further towards the blade as compared to the tissue-contacting surface of the jaw liner.

In still yet another aspect of the present disclosure, the blade includes a tissue-contacting surface having a convex configuration defining an apex, and the jaw includes a structural body and a jaw liner. The jaw liner defines a tissue-contacting surface and the structural body defines first and second tissue-contacting surfaces disposed on either side of the tissue-contacting surface of the jaw liner. In such aspects, in the approximated position, the tissue-contacting surface of the blade and the tissue-contacting surface of the jaw liner are configured to grasp a center portion of tissue therebetween, and the first and second tissue-contacting surfaces of the structural body and the first and second panels, respectively, are configured to grasp outer lateral portions of tissue therebetween.

In another aspect of the present disclosure, the first and second panels are fixed relative to the blade. Alternatively, the first and second panels are movable relative to the blade between a more-proximal position and a more-distal position. In such movable aspects, the first and second panels may be movable relative to the blade between the more-proximal position and the more-distal position in connection with pivoting of the jaw between the spaced-apart position and the approximated position.

In yet another aspect of the present disclosure, the blade is curved and the first and second panels are similarly curved.

In still another aspect of the present disclosure, the housing further supports an ultrasonic generator configured to drive the ultrasonic transducer. In such aspects, the housing may further support a battery assembly configured to power the ultrasonic generator.

An exemplary method not forming part of the invention of sealing and cutting tissue provided in accordance with aspects of the present disclosure includes clamping tissue with an end effector including an ultrasonic blade, first and second panels disposed on opposite sides of the ultrasonic blade in laterally-spaced relation relative thereto and extending along at least a portion of a length of the ultrasonic blade, and a jaw. Tissue is clamped such that the ultrasonic blade and the jaw define a first distance therebetween and grasp a center portion of tissue therebetween under a first clamping force, and such that the jaw and each of the first and second panels define a second distance therebetween greater than the first distance and grasp outer lateral portions of tissue therebetween under a second clamping pressure less than the first clamping pressure. In other aspects, the second distance may be substantially equal to the first distance or less than the first distance such that the second clamping pressure is substantially equal to or greater than the first clamping pressure.

The exemplary method further includes activating the ultrasonic blade to heat the center portion of tissue to a first temperature and cut the center portion of tissue, and heat the outer lateral portions of tissue to a second temperature less than the first temperature and seal the outer lateral portions of tissue.

In an aspect of the present disclosure, clamping the tissue includes applying a first compression to the center portion of tissue and applying a second compression to the outer lateral portions of tissue that is less than the first compression.

In another aspect of the present disclosure, clamping the tissue includes moving the jaw from a spaced-apart position to an approximated position relative to the ultrasonic blade and the first and second panels. Moving the jaw from the spaced-apart position to the approximated position, in aspects, may effect deployment of the first and second panels from a more-proximal position to a more-distal position.

In yet another aspect of the present disclosure, the center portion of tissue is clamped between the ultrasonic blade and a jaw liner of the jaw, and wherein the outer lateral portions of tissue are clamped between a structural body of the jaw and the first and second panels.

In still another aspect of the present disclosure, activating the ultrasonic blade includes driving an ultrasonic transducer to transmit ultrasonic energy along a waveguide to the ultrasonic blade.

Also provided in accordance with aspects of the present disclosure is an ultrasonic surgical system including an ultrasonic generator, a housing, an ultrasonic transducer supported by the housing, a waveguide coupled to the ultrasonic transducer, the waveguide defining a blade at a distal end portion thereof, and first and second panels disposed on opposite sides of the blade in laterally-spaced relation relative thereto. The ultrasonic generator is electrically coupled to the ultrasonic transducer for transmitting an ultrasonic drive signal to the ultrasonic transducer for driving the ultrasonic transducer to transmit ultrasonic energy along the waveguide to the blade.

The ultrasonic generator is also electrically coupled to the blade and the first and second panels for transmitting an interrogation signal between the blade, the first and second panels, and tissue.

In an aspect of the present disclosure, the interrogation signal is a Radio Frequency (RF) signal. In such aspects, the blade may be configured to define a first potential and the first and second panels may be configured to define a second, different potential for transmitting the RF signal therebetween and through tissue.

In another aspect of the present disclosure, the ultrasonic generator is configured to alternatingly or simultaneously transmit the ultrasonic drive signal and the interrogation signal.

In another aspect of the present disclosure, the ultrasonic drive signal includes an AC waveform. Alternatively or additionally, the interrogation signal includes at least one pulse.

In still another aspect of the present disclosure, the ultrasonic generator is configured to determine tissue impedance based upon the interrogation signal.

In yet another aspect of the present disclosure, the ultrasonic surgical system further includes a jaw pivotable relative to the blade and the first and second panels between a spaced-apart position and an approximated position.

In still yet another aspect of the present disclosure, the housing further supports the ultrasonic generator. In such aspects, the housing may also support a battery assembly configured to power the ultrasonic generator.

An exemplary method of treating tissue, not forming part of the invention, provided in accordance with aspects of the present disclosure includes transmitting an ultrasonic drive signal from an ultrasonic generator to an ultrasonic transducer to drive the ultrasonic transducer to transmit ultrasonic energy along a waveguide to a blade. The method further includes transmitting an interrogation signal from the ultrasonic generator to the blade, through tissue, to first and second panels disposed on opposite sides of the blade in laterally-spaced relation relative thereto, and back to the ultrasonic generator to determine at least one parameter of tissue.

In an aspect of the present disclosure, transmitting the interrogation signal includes defining a first potential at the blade and defining a second, different potential at the first and second panels to transmit an RF signal therebetween and through tissue.

In another aspect of the present disclosure, transmitting the ultrasonic drive signal includes transmitting an AC waveform and/or transmitting the interrogation signal includes transmitting at least one pulse.

In yet another aspect of the present disclosure, the method further includes stopping transmission of the ultrasonic drive signal before transmitting the interrogation signal and resuming transmission of the ultrasonic drive signal after transmitting the interrogation signal.

In still another aspect of the present disclosure, the ultrasonic generator is configured to alternatingly transmit the ultrasonic drive signal and the interrogation signal.

In another aspect of the present disclosure, determining the at least one parameter of tissue includes determining tissue impedance.

In another aspect of the present disclosure, the method further includes providing at least one output in response to determining the at least one parameter of tissue.

In still yet another aspect of the present disclosure, the method further includes modifying the ultrasonic drive signal in response to determining the at least one parameter of tissue.

A surgical end effector assembly provided in accordance with the present disclosure and configured for use with any of the instruments or systems detailed herein or other suitable instruments or systems includes an ultrasonic blade adapted to connect to an ultrasonic transducer configured to transmit ultrasonic energy to the ultrasonic blade, first and second panels disposed on opposite sides of the ultrasonic blade in laterally-spaced relation relative thereto, and a jaw pivotable relative to the ultrasonic blade and the first and second panels between open and clamping positions. At least two of the ultrasonic blade, the first and second panels, or the jaw are energizable to different electrical potentials for transmitting an electric (e.g., a Radio Frequency (RF) or a Direct Current (DC)) signal therebetween and through tissue for at least one of interrogating tissue or treating tissue.

In an aspect of the present disclosure, the ultrasonic blade is configured to be energized to a first potential and the first and second panels are configured to be energized to a second, different potential for transmitting the electric signal therebetween and through tissue.

In another aspect of the present disclosure, the jaw is configured to be energized to a first potential and the first and second panels are configured to be energized to a second, different potential for transmitting the electric signal therebetween and through tissue.

In yet another aspect, the jaw includes a structural body defining first and second surfaces, and a jaw liner disposed between the first and second surfaces. In such aspects, the first and second surfaces are energizable for transmitting the electric signal.

In still another aspect of the present disclosure, the jaw includes a structural body supporting first and second electrically-conductive plates, and a jaw liner disposed between the first and second electrically-conductive plates. In such aspects, the first and second electrically-conductive plates are energizable for transmitting the electric signal.

In still yet another aspect of the present disclosure, an insulative coating is disposed on at least one of outer lateral sides of the ultrasonic blade or inner lateral sides of the first and second panels.

In another aspect of the present disclosure, a gap distance is maintained between outer lateral sides of the ultrasonic blade and inner lateral sides of the first and second panels.

In still another aspect of the present disclosure each of the first and second panels includes a vertical body and a shelf extending laterally from the vertical body. The shelf defines an opposing surface positioned to oppose the jaw in the clamping position of the jaw.

In yet another aspect of the present disclosure, each of the first and second panels tapers in height in a proximal-to-distal direction from lower portions thereof to increasingly expose a bottom portion of the blade in a proximal-to-distal direction.

Another surgical end effector assembly provided in accordance with the present disclosure and configured for use with any of the instruments or systems detailed herein or other suitable instruments or systems includes an ultrasonic blade adapted to connect to an ultrasonic transducer configured to transmit ultrasonic energy to the ultrasonic blade, first and second panels disposed on opposite sides of the ultrasonic blade in laterally-spaced relation relative thereto, and a jaw pivotable relative to the ultrasonic blade and the first and second panels between open and clamping positions. The jaw includes a structural body, a jaw liner supported by the structural body and positioned to oppose the ultrasonic blade in the clamping position of the jaw, and first and second energy elements supported by the structural body on either side of the jaw liner and positioned to oppose the first and second panels, respectively, in the clamping position of the jaw. The first and second energy elements are configured to facilitate delivery of another energy to tissue disposed between the first and second energy elements and the first and second panels, respectively. The another energy may be different from ultrasonic energy.

In an aspect of the present disclosure, the first and second energy elements are optical emitters configured to emit optical energy through tissue disposed between the first and second energy elements and the first and second panels, respectively. In such aspects, the first and second panels may be configured as optical absorbers or optical reflectors.

In another aspect of the present disclosure, the first and second energy elements are ultrasound transducers configured to emit ultrasound wave energy through tissue disposed between the first and second energy elements and the first and second panels, respectively. In such aspects, the first and second panels may be configured as ultrasound absorbers or ultrasound reflectors.

In yet another aspect of the present disclosure, the first and second energy elements are thermal heaters configured to conduct thermal energy to tissue disposed between the first and second energy elements and the first and second panels, respectively.

In still another aspect of the present disclosure, the another energy is utilized to treat tissue and/or to interrogate tissue.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1A is a side, perspective view of an ultrasonic surgical instrument provided in accordance with the present disclosure;
FIG. 1B is a perspective view of an ultrasonic surgical system provided in accordance with the present disclosure including another ultrasonic surgical instrument;
FIG. 2 is an enlarged, side, longitudinal, cross-sectional view of a proximal portion of the ultrasonic surgical instrument of FIG. 1A;
FIG. 3 is an enlarged, perspective view of a distal portion of the ultrasonic surgical instrument of FIG. 1A illustrating an end effector assembly thereof;
FIG. 4 is an enlarged, side view of the distal portion of the ultrasonic surgical instrument of FIG. 1A;
FIG. 5 is a schematic illustration of the end effector assembly of the ultrasonic surgical instrument of FIG. 1A in use sealing and cutting tissue;
FIG. 6 is a schematic illustration of the end effector assembly and generator of the ultrasonic surgical instrument of FIG. 1A in use interrogating tissue;
FIGS. 7A-7D are graphical illustrations representing generator signals as a function of time in accordance with aspects of the present disclosure;
FIG. 8 is a schematic illustration of another end effector assembly in accordance with the present disclosure configured for applying ultrasonic and Radio Frequency (RF) energy;
FIG. 9 is a schematic illustration of still another end effector assembly in accordance with the present disclosure configured for applying ultrasonic and RF energy;
FIG. 10 is a schematic illustration of yet another end effector assembly in accordance with the present disclosure configured for applying ultrasonic and RF energy;
FIGS. 11A and 11B are schematic illustrations of still yet other end effector assemblies in accordance with the present disclosure configured for applying ultrasonic and RF energy;
FIG. 12 is a transverse, cross-sectional view illustrating a tube set arrangement in accordance with the present disclosure configured for use with a surgical instrument or system configured for applying ultrasonic and RF energy;
FIGS. 13A and 13B are schematic illustrations of other end effector assemblies in accordance with the present disclosure configured for applying ultrasonic and optical energy;
FIG. 14 is a schematic illustration of another end effector assembly in accordance with the present disclosure configured for applying ultrasonic and ultrasound energy;
FIG. 15 is a schematic illustration of yet another end effector assembly in accordance with the present disclosure configured for applying ultrasonic and thermal energy;
FIG. 16A is a side view of another end effector assembly configured for use with an ultrasonic surgical instrument or system;
FIG. 16B is a front, perspective view of the end effector assembly of FIG. 16A;
FIGS. 17A, 17B, and 17C are side, front perspective, and transverse, cross-sectional views of an end effector assembly configured for use with a robotic surgical; and
FIGS. 18A, 18B, and 18C are side, front perspective, and transverse, cross-sectional views of another end effector assembly configured for use with a robotic surgical system.

### DETAILED DESCRIPTION

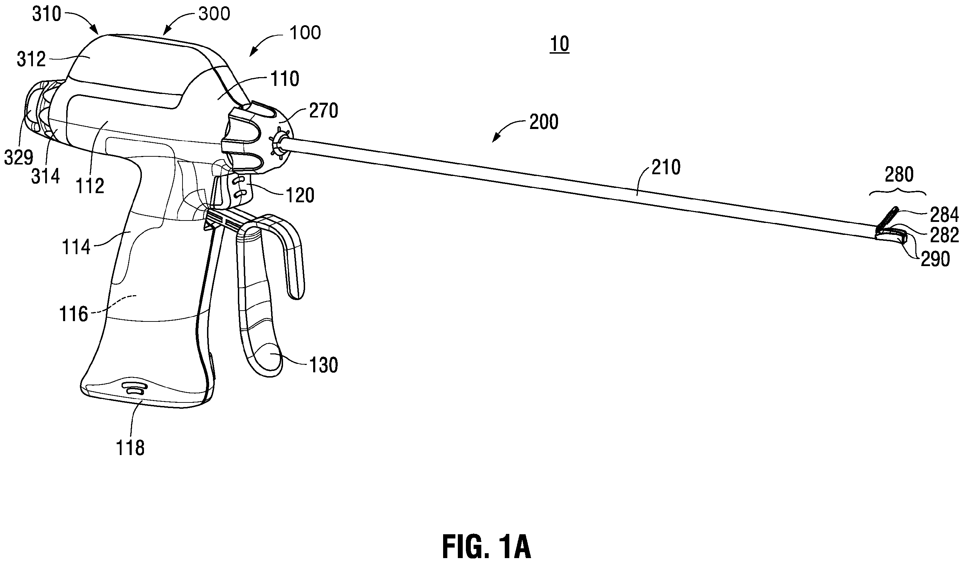

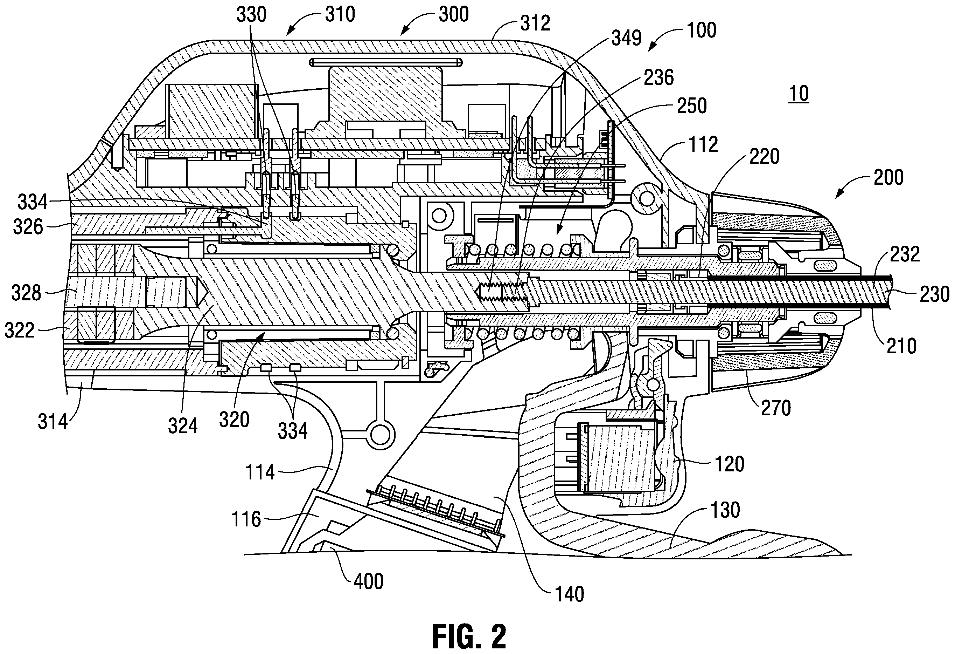

Referring to FIGS. 1A and 2, an ultrasonic surgical instrument 10 provided in accordance with the present disclosure includes a handle assembly 100 and an elongated assembly 200 extending distally from handle assembly 100. Handle assembly 100 includes a housing 110 defining a body portion 112 and a fixed handle portion 114. Handle assembly 100 further includes an activation button 120 and a clamp trigger 130.

Body portion 112 of housing 110 is configured to support an ultrasonic transducer and generator assembly ("TAG") 300 including a generator 310 and an ultrasonic transducer 320. TAG 300 may be permanently engaged with body portion 112 of housing 110 or removable therefrom. Generator 310 includes a housing 312 configured to house the internal electronics of generator 310, and a cradle 314 configured to rotatably support ultrasonic transducer 320.

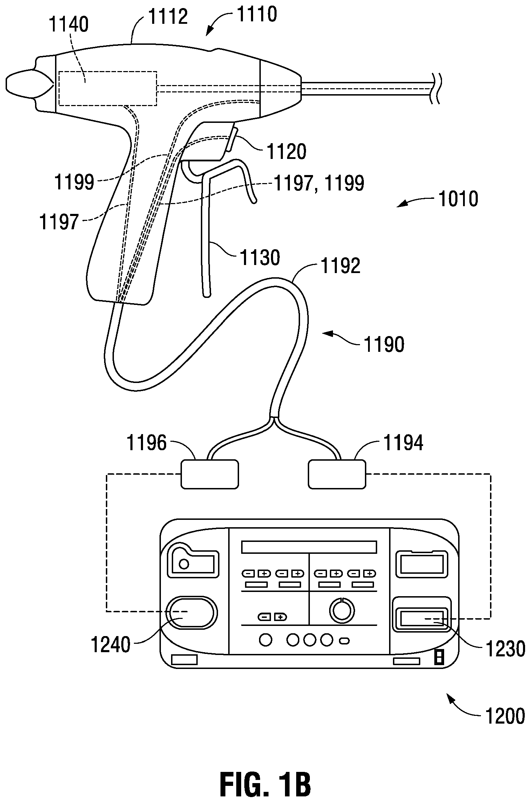

Continuing with reference to FIGS. 1A and 2, ultrasonic transducer 320 includes a piezoelectric stack 322, a horn 324, a casing 326, and a bolt 328 securing piezoelectric stack 322 between horn 324 and a proximal nut (not shown). Ultrasonic transducer 320 further includes a rotation knob 329 (FIG. 1). Casing 326 and rotation knob 329 are engaged with one another and cooperate to form an enclosure to encapsulate the proximal nut, piezoelectric stack 322, and a portion of horn 324, with the remainder of horn 324 extending distally from casing 326. Rotation knob 329 is accessible from the exterior of handle assembly 100 and is configured for manual rotation to rotate ultrasonic transducer 320 relative to generator 310 and housing 110.

A set of connectors 330 and corresponding rotational contacts 334 associated with generator 310 and ultrasonic transducer 320, respectively, enable ultrasonic drive signals to be communicated from generator 310 to piezoelectric stack 322 of ultrasonic transducer 320 to drive ultrasonic transducer 320 regardless of the rotational orientation of ultrasonic transducer 320 relative to generator 310. Horn 324, in turn, is configured to transmit the ultrasonic energy produced by piezoelectric stack 322 to waveguide 230 of elongated assembly 200 for transmission therealong to blade 282 of end effector 280 of elongated assembly 200, as detailed below.

Referring still to FIGS. 1A and 2, fixed handle portion 114 of housing 110 defines a compartment 116 configured to receive a battery assembly 400 and a door 118 configured to enclose compartment 116. An electrical connection assembly 140 is disposed within housing 110 of handle assembly 100 and serves to electrically couple activation button 120, generator 310 of TAG 300, and battery assembly 400 with one another when TAG 300 is supported on or within body portion 112 of housing 110 and battery assembly 400 is disposed within compartment 116 of fixed handle portion 114 of housing 110, thus enabling activation of ultrasonic surgical instrument 10 in response to depression of activation button 120. In embodiments, activation button 120 may include multiple actuated stages or multiple activation buttons may be provided to enable activation in either a LOW power mode or a HIGH power mode. In embodiments where generator 310 is remote from ultrasonic surgical instrument 10, battery assembly 400 and the configuration of fixed handle portion 114 for receiving battery assembly 400 need not be provided, as generator 310 may be powered by a standard wall outlet or other power source.

Referring to FIG. 1B, a surgical system provided in accordance with aspects of the present disclosure is shown generally identified by reference numeral 1010 including a surgical instrument 1100 and a surgical generator 1200. Surgical system 1010 generally differs from surgical instrument 10 (FIG. 1A) in that, rather than providing an on-board generator, e.g., generator 310 of TAG 300 (FIG. 1A), and power source, e.g., battery assembly 400 (FIG. 1A), surgical system 1010 includes surgical instrument 1100 and remotely-disposed surgical generator 1200 connected by way of a cable and plug assembly 1190. Surgical generator 1200 is adapted to connect to a power source (not shown), e.g., a standard wall outlet. As surgical system 1010 may otherwise be similar to surgical instrument 10 (FIG. 1A), only differences therebetween are described in detail below while similarities are summarily described or omitted entirely. Further, unless explicitly contradicted, it is understood that aspects and features of the present disclosure made with reference to surgical instrument 10 (FIG. 1A) may likewise apply to surgical system 1010.

Surgical generator 1200 of surgical system 1010 includes an ultrasonic plug port 1230, and a Radio Frequency (RF) plug port 1240. Surgical generator 1200 is configured to produce ultrasonic drive signals for output through ultrasonic plug port 1230 to surgical instrument 1100 to activate surgical instrument 1100 in an ultrasonic mode of operation and to provide RF energy for output through RF plug port 1240 to surgical instrument 1100 to activate surgical instrument 1100 in an RF mode of operation, e.g., for treating tissue and/or sensing one or more parameters of tissue and/or surgical instrument 1100. It is also contemplated that a common port (not shown) configured to act as both ultrasonic plug port 1230 and an RF plug port 1240 may be utilized.

Surgical instrument 1010 includes a handle assembly 1110, an elongated assembly 1150 extending distally from handle assembly 1110, and cable and plug assembly 1190 which is operably coupled with handle assembly 1110 and extends therefrom for connection to surgical generator 1200. Elongated assembly 1150 is similar to elongated assembly 200 (FIG. 1A), detailed below, and may include any of the features thereof. Handle assembly 1110 is similar to handle assembly 100 (FIG. 1A), as detailed above, except that, rather than receiving a TAG 300 (FIG. 1A), the body portion of handle housing 1112 is only configured to support an ultrasonic transducer 1140. Ultrasonic transducer 1140 may be permanently engaged with the body portion of housing 1112 or removable therefrom. Ultrasonic transducer 1140 includes a piezoelectric stack or other suitable ultrasonic transducer components electrically coupled to surgical generator 1200, e.g., via one or more of first electrical lead wires 1197, to enable communication of ultrasonic drive signals to ultrasonic transducer 1140 to drive ultrasonic transducer 1140 to produce ultrasonic vibration energy that is transmitted along a waveguide to a blade for treating tissue therewith.

Activation button 1120 of handle assembly 1100 is coupled to or between ultrasonic transducer 1140 and/or surgical generator 1200, e.g., via one or more of first electrical lead wires 1197, to enable activation of ultrasonic transducer 1140 in response to depression of activation button 1120. In embodiments, activation button 1120 may be an ON/OFF switch. In other embodiments, activation button 1120 may include multiple actuated stages to enable activation from an OFF position to different actuated positions corresponding to different modes, e.g., a first actuated position corresponding to a first mode and a second actuated position corresponding to a second mode. In still other embodiments, separate activation buttons may be provided, e.g., a first actuation button for activating a first mode and a second activation button for activating a second mode.

Continuing with reference to FIG. 1B, cable and plug assembly 1190 of surgical instrument 1100 includes a cable 1192, an ultrasonic plug 1194, and an RF plug 1196. Ultrasonic plug 1194 is configured for connection with ultrasonic plug port 1230 of surgical generator 1200 while RF plug 1196 is configured for connection with electrosurgical plug port 1240 of surgical generator 1200. In embodiments where generator 1200 includes a common port, plug assembly 1190 may include a common plug (not shown) configured to act as both the ultrasonic plug 1194 and the RF plug 1196. Plural first electrical lead wires 1197 electrically coupled to ultrasonic plug 1194 extend through cable 1192 and into handle assembly 1110 for electrical connection to ultrasonic transducer 1140 and/or activation button 1120 to enable the selective supply of ultrasonic drive signals from surgical generator 1200 to ultrasonic transducer 1140 upon activation of activation button 1120 in an ultrasonic mode of operation. In addition, plural second electrical lead wires 1199 are electrically coupled to RF plug 1196 and extend through cable 1192 into handle assembly 1110. One or more second electrical lead wires 1199 is electrically coupled to activation button 1120 to enable the selective supply of RF energy from surgical generator 1200 in an RF mode of operation, as detailed below.

With reference to FIGS. 1A, 2, 3 and 4, elongated assembly 200 of ultrasonic surgical instrument 10 includes an outer drive sleeve 210, an inner support sleeve 220 disposed within outer drive sleeve 210, a waveguide 230 extending through inner support sleeve 220, a drive assembly 250, a rotation knob 270, and an end effector 280 including a blade 282, a jaw 284, and a pair of panels 290. A proximal portion of outer drive sleeve 210 is operably coupled to clamp trigger 130 of handle assembly 100 via drive assembly 250, while a distal portion of outer drive sleeve 210 is operably coupled to jaw 284. As such, clamp trigger 130 is selectively actuatable to thereby move outer drive sleeve 210 about inner support sleeve 220 to pivot jaw 284 relative to blade 282 of end effector 280 from a spaced-apart position to an approximated position for clamping tissue between jaw 284 and blade 282. Drive assembly 250 may provide a forcelimiting feature whereby the clamping pressure applied to tissue clamped between jaw 284 and blade 282 is limited to a particular clamping pressure or particular clamping pressure range. Rotation knob 270 is rotatable in either direction to rotate elongated assembly 200 in either direction relative to handle assembly 100.

Waveguide 230, as noted above, extends through inner support sleeve 220. Waveguide 230 defines a body 232 and blade 282 extending from the distal end of body 232. Waveguide 230 (including blade 282) may be formed from titanium, a titanium alloy, or other suitable material(s). Blade 282 serves as the blade of end effector 280 and may be integrally formed with waveguide 230 or separately formed and subsequently attached (permanently or removably) to waveguide 230. Waveguide 230 further includes a proximal threaded male connector 236 configured for threaded engagement within threaded female receiver 349 of horn 324 such that ultrasonic motion produced by ultrasonic transducer 320 is transmitted along waveguide 230 to blade 282 for treating tissue clamped between blade 282 and jaw 284 or positioned adjacent to blade 282.

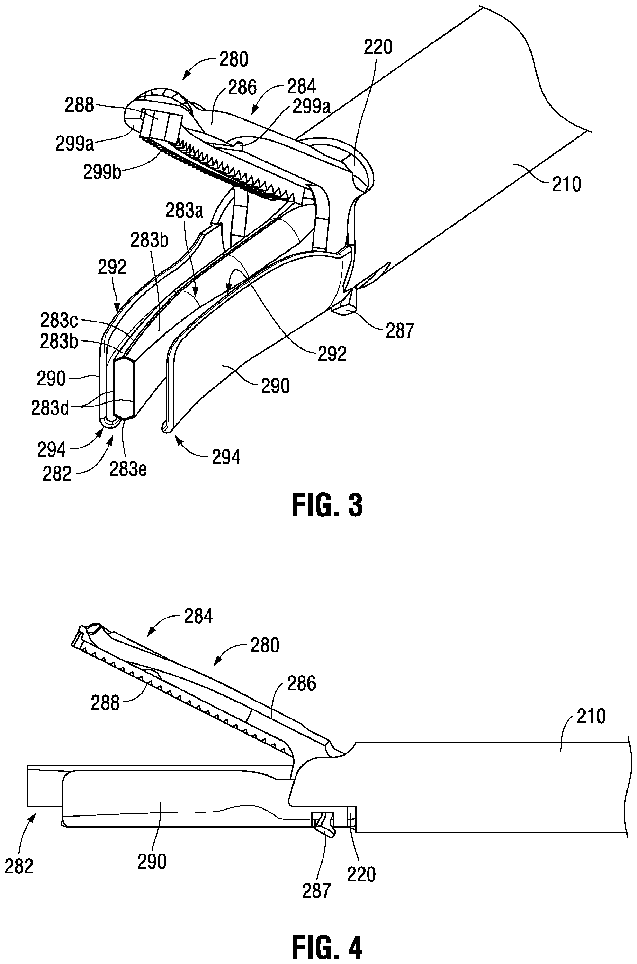

Referring in particular to FIGS. 3 and 4, blade 282 of end effector 280 defines a curved configuration, although straight configurations are also contemplated. Blade 282 may be curved in any direction relative to jaw 284, for example, such that the distal tip of blade 282 is curved towards jaw 284, away from jaw 284, or laterally (in either direction) relative to jaw 284. Further, blade 282 may be formed to include multiple curves in similar directions, multiple curves in different directions within a single plane, and/or multiple curves in different directions in different planes. Blade 282 may additionally or alternatively be formed to include a tapered configuration, various different cross-sectional configurations along its length, cut-outs, indents, edges, protrusions, straight surfaces, curved surfaces, angled surfaces, wide edges, narrow edges, and/or other features.

In embodiments, blade 282 defines a generally convex first tissue-contacting surface 283a, e.g., the surface that opposes jaw 284. Generally convex first tissue-contacting surface 283a may defined by a pair of surfaces 283b (flat or arcuate surfaces) that converge at an apex 283c, or may be formed by a single arcuate surface defining an apex 283c. Blade 282 may further define substantially flat lateral surfaces 283d (excluding any curvature due to the curvature of blade 282 itself) on either side of first tissue-contacting surface 283a, and a second tissue-contacting surface 283e opposite first tissue-contacting surface 283a and similarly configured relative thereto, although other configurations are also contemplated. First tissue-contacting surface 283a is configured to contact tissue clamped between blade 282 and jaw 284 for, e.g., sealing and cutting clamped tissue, while second tissue-contacting surface 283e may be utilized for, e.g., tissue dissection, back scoring, etc.

Jaw 284 of end effector 280 includes a more-rigid structural body 286 and a morecompliant jaw liner 288. Structural body 286 includes a pair of proximal flanges 287 that are pivotably coupled to inner support sleeve 220 and operably associated with outer drive sleeve 210, e.g., via receipt within apertures defined within outer drive sleeve 210, such that sliding of outer drive sleeve 210 about inner support sleeve 220 pivots jaw 284 relative to blade 282 from a spaced-apart position to an approximated position to clamp tissue between jaw liner 288 of jaw 284 and blade 282.

Jaw liner 288 of jaw 284 may be fabricated from a compliant material such as, for example, polytetrafluoroethylene (PTFE), such that blade 282 is permitted to vibrate while in contact with jaw liner 288 without damaging components of ultrasonic surgical instrument 10 (FIG. 1), e.g., structural body 286 of jaw 284, and without compromising the hold on tissue clamped between jaw 284 and blade 282. Other suitable materials are also contemplated. Jaw liner 288 may be monolithically or otherwise formed.

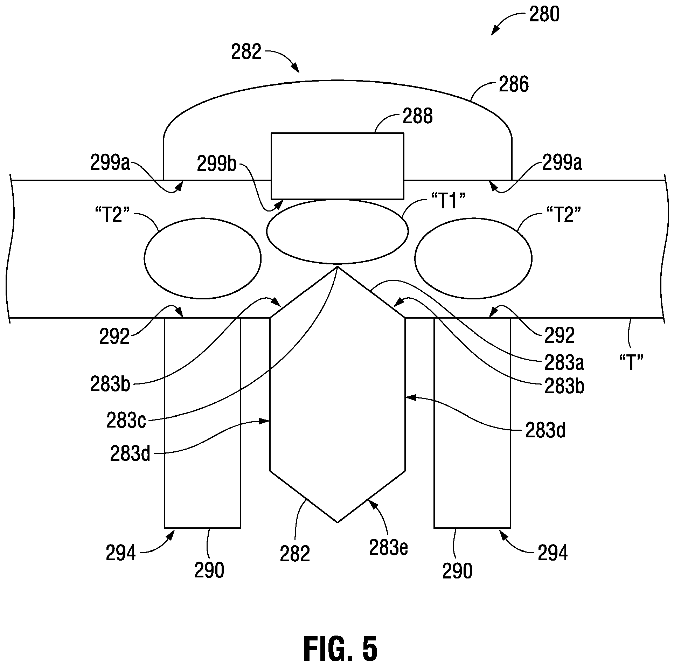

In embodiments, as illustrated in FIGS. 3 and 4, jaw liner 288 protrudes from structural body 286 such that tissue-contacting surfaces 299a of structural body 286, disposed on on either side of jaw liner 288, are recessed relative to tissue-contacting surface 299b of jaw liner 288 (see also FIG. 5). Further, jaw liner 288 defines a width that approximates or is greater than a width of blade 282 to enable tissue to be clamped between tissue-contacting surface 299b of jaw liner 288 and blade 282 across the full width of blade 282.

Panels 290 extend distally from outer and inner sleeves 210, 220 on either side of blade 282 in laterally-spaced relation relative thereto such that the spaces between panels 290 and blade 282, e.g., between lateral surfaces 283d of blade 282, form thermally-insulating air gaps therebetween. Panels 290 define relatively thin, plate-like configurations disposed in vertical orientation such that the height dimensions thereof extend parallel relative to an axis of motion of jaw 284 between the spaced-apart and approximated positions, and such that the width dimensions thereof extend perpendicular relative to the axis of motion of jaw 284 between the spaced-apart and approximated positions. In this manner, panels 290 define relatively narrow tissue-contacting surfaces 292. In embodiments, panels 290 are flared outwardly away from blade 282 or inwardly towards blade 282 whereby the flared portions of panels 290 define tissue-contacting surfaces 292 of greater surface area. In such embodiments, the flared portions of panels 290 may extend generally perpendicularly relative to the vertical body portions of panels 290 such that tissue-contacting surfaces 292 are generally parallel with tissue-contacting surfaces 299a of structural body 286. Other configurations are also contemplated.

Panels 290, in embodiments, are positioned such that blade 282 is laterally centered relative to panels 290, e.g., wherein panels 290 are evenly spaced-apart from blade 282 on either side thereof. Further, in embodiments where blade 282 defines a laterally-curved configuration, panels 290 may likewise define similar curvatures to track the curvature of blade 282 and maintain a substantially uniform spacing therebetween, although other configurations are also contemplated. Alternatively, panels 290 may be positioned such that a uniform gap between blade 282 and the corresponding panel 290 on the concave side of blade 282 is smaller than a uniform gap between blade 282 and the corresponding pane 290 on the convex side of blade 282. In embodiments, protrusions or other features (not shown) extend from panels 290 to contact blade 282, e.g., at a node point, in order to maintain appropriate spacing between panels 290 and blade 282. Panels 290 may be formed from stainless steel or other suitable material(s).

In embodiments, panels 290 define heights that are equal to or greater than the heights of lateral surfaces 283d of blade 282 but equal to or less than the overall height of blade 282, e.g., from the apex 283c of first tissue-contacting surface 283a to the apex of second tissue-contacting surface 283e. More specifically, in embodiments, panels 290 are vertically positioned relative to blade 282 such that tissue-contacting surfaces 292 of panels 290 are horizontally-aligned with the upper ends of lateral surfaces 283d of blade 282. As such, at least a portion of first tissue-contacting surface 283a of blade 282 extends above panels 290 towards jaw 284. The opposite surfaces 294 of panels 290 may be horizontally-aligned with the lower ends of lateral surfaces 283d of blade 282, may be horizontally-aligned with the apex of second tissue-contacting surface 283e of blade 282 (as illustrated (see FIG. 5)), may be disposed between with the lower ends of lateral surfaces 283d of blade 282 and the apex of second tissue-contacting surface 283e, or may extend beyond the apex of second tissue-contacting surface 283e.

Panels 290 are spaced-apart from one another a distance greater than the width of tissue-contacting surface 299b of jaw liner 288 such that, in the approximated position of jaw 284, no portion of tissue-contacting surface 299b of jaw liner 288 directly opposes the tissue-contacting surface 292 of either of panels 290 (see also FIG. 5). However, panels 290 are spaced-apart from one another a distance less than the width of structural body 286 such that, in the approximated position of jaw 284, in addition to clamping tissue between first tissue-contacting surface 283a of blade 282 and tissue-contacting surface 299b of jaw liner 288, tissue on either side thereof is clamped between tissue-contacting surfaces 292 of panels 290 and tissue-contacting surfaces 299a of structural body 286, albeit with a greater distance between tissue-contacting surfaces 292 and tissue-contacting surfaces 299a as compared to the distance (or maximum distance) between tissue-contacting surface 299b of jaw line 288 and first tissue-contacting surface 283a of blade 282. Alternatively, the greater distance between tissue-contacting surfaces 292 and tissue-contacting surfaces 299a may be substantially equal to or less than the distance (or maximum distance) between tissue-contacting surface 299b of jaw line 288 and first tissue-contacting surface 283a of blade 282.

Continuing with reference to FIGS. 3 and 4, panels 290 may be fixedly engaged with and extend from inner support sleeve 220 such that panels 290 are fixed in position relative to blade 282 (aside from the ultrasonic vibration of blade 282 during activation) and extend along at least a portion of the length of blade 282. In embodiments, blade 282 extends distally beyond the distal ends of panels 290 to enable use of the exposed distal portion of blade 282 for tissue dissection or other purposes without obstruction from panels 290. In such embodiments, panels 290 may serve as a depth-stop inhibiting blade 282 from dissecting too far through tissue, e.g., beyond the distance blade 282 extends distally from panels 290.

As an alternative to panels 290 being fixed relative to blade 282, panels 290 may be fixedly engaged with and extend from outer drive sleeve 210 such that panels 290 are movable between a more-proximal position, wherein panels 290 do not extend to blade 282 or extend along a relatively smaller portion of the length of blade 282, and a more-distal position, wherein panels 290 extend along the entire or a relatively greater portion of the length of blade 282, in response to translation of outer drive sleeve 210, e.g., via squeezing clamp trigger 130 towards fixed handle portion 114 (see FIG. 1). As translation of outer drive sleeve 210 also effects pivoting of jaw 284 relative to blade 282, the more-proximal position may correspond to the spaced-apart position of jaw 284, while the more-distal position corresponds to the approximated positions of jaw 284.

In embodiments, panels 290 are engaged with a third sleeve (not shown), e.g., disposed between outer and inner sleeves 210, 220, respectively, to enable movement of panels 290 between the more-proximal and more-distal positions. This may be accomplished independently of the pivoting of jaw 284, e.g., via a separate actuator (not shown) disposed on handle assembly 100 (FIG. 1), or in cooperation therewith, e.g., wherein the third sleeve is coupled to clamp trigger 130 (FIG. 1). In embodiments where the third sleeve is coupled to clamp trigger 130 (FIG. 1), this may be accomplished via one of the differential deployment mechanisms detailed in U.S. Patent No. 9,375,205 to Mueller, or other suitable mechanism, to enable movement of outer drive sleeve 220 and panels 290 different distances in response to actuation of clamp trigger 130 (FIG. 1).

Referring generally to FIGS. 1-4, in use, ultrasonic instrument 10 is advanced into a surgical site and manipulated, e.g., end effector 280 is rotated via rotation of rotation knob 329 and/or rotation knob 270, such that end effector 280 is positioned with tissue to be treated disposed between jaw 284 and blade 282 with jaw 284 disposed in the spaced-apart position (FIG. 1). Thereafter, clamp trigger 130 is squeezed towards fixed handle portion 114 of housing 110 from an un-actuated position to an actuated position to translate outer drive sleeve 210 about inner support sleeve 220 and relative to end effector 280, thereby pivoting jaw 284 relative to blade 282 from the spaced-apart position towards the approximated position to clamp tissue between jaw 284 and blade 282 (and between panels 290 and jaw 284). Panels 290 may facilitate the clamping, retention and manipulation of tissue "T" and inhibit slippage thereof by providing additional surface area with which to clamp.

Turning to FIG. 5, the tissue "T" grasped with end effector 280 can generally be characterized as including three portions: a center portion of tissue "T1" and a pair of outer lateral portions of tissue "T2" disposed on either side of the center portion of tissue "T1." The center portion of tissue "T1" is clamped between first tissue-contacting surface 283a of blade 282 and tissue-contacting surface 299b of jaw liner 288 of jaw 284, wherein apex 283c facilitates application of relatively higher compression on the center portion of tissue "T1" and the configuration of first tissue-contacting surface 283a of blade 282 and tissue-contacting surface 299b of jaw liner 288 being relatively closer-together provides a relatively higher clamping force on the center portion of tissue "T1." The outer lateral portions of tissue "T2," on the other hand, are clamped between tissue-contacting surfaces 299a of structural body 286 of jaw 284 and tissue-contacting surfaces 292 of panels 290. Due to the relatively flat configuration of tissue-contacting surfaces 292 of panels and the configuration of tissue-contacting surfaces 299a and tissue-contacting surfaces 292 being relatively farther-apart, the compression and clamping force applied to the outer lateral portions of tissue "T2" are relatively lower. Of course, as the tissue "T" is a continuous mass, portions of tissue also extend between the portions "T1" and "T2." As an alternative to the above, the outer lateral portions of tissue "T2" may be clamped with relatively higher clamping forces as compared to the clamping force applied to the center portion of tissue "T1."

With tissue clamped in the manner detailed above, blade 282 may be activated, e.g., via depression of activation button 120 (FIG. 1). Referring also to FIGS. 1 and 2, upon depression of activation button 120, a DC power signal is supplied from battery assembly 400 to generator 310 which converts the DC power signal into a high voltage AC waveform ultrasonic drive signal that is transmitted to piezoelectric stack 324 of ultrasonic transducer 320. Activation of piezoelectric stack 324 in this manner produces ultrasonic energy that is transmitted to ultrasonic horn 326, along waveguide 230, and to blade 282 such that blade 282 is ultrasonically vibrated. The ultrasonic energy provided at blade 282 is used to heat adjacent tissue "T" to seal and cut the tissue "T." More specifically, during activation, due to the direct contact of the center portion of tissue "T1" with blade 282 and the relatively higher compression and clamping force on the center portion of tissue "T1," the center portion of tissue "T1" is heated to a relatively higher temperature and is dissected, e.g., cut, thereby separating the outer lateral portions of tissue "T2" from one another. Although some sealing of the center portion of tissue "T1" may be accomplished, the majority of the tissue effect on the center portion of tissue "T1" is cutting.

Also during activation, the ultrasonic energy provided at blade 282 heats the outer lateral portions of tissue "T2." However, this is indirect heating given that the outer lateral portions of tissue "T2" are laterally displaced from blade 282. This, coupled with the relatively lower compression and clamping force on the outer lateral portions of tissue "T2," results in heating of the outer lateral portions of tissue "T2" to a relatively lower temperature and sealing of the outer lateral portions of tissue "T2." Although some cutting of the outer lateral portions of tissue "T2" may be accomplished, the majority of the tissue effect on the outer lateral portions of tissue "T2" is sealing. The result of the above is that tissue "T" is sealed in two laterally spaced-apart locations, e.g., at both portions "T2," and cut therebetween, e.g., at portion "T1." Panels 290, in embodiments, may also help limit thermal spread laterally therebeyond.

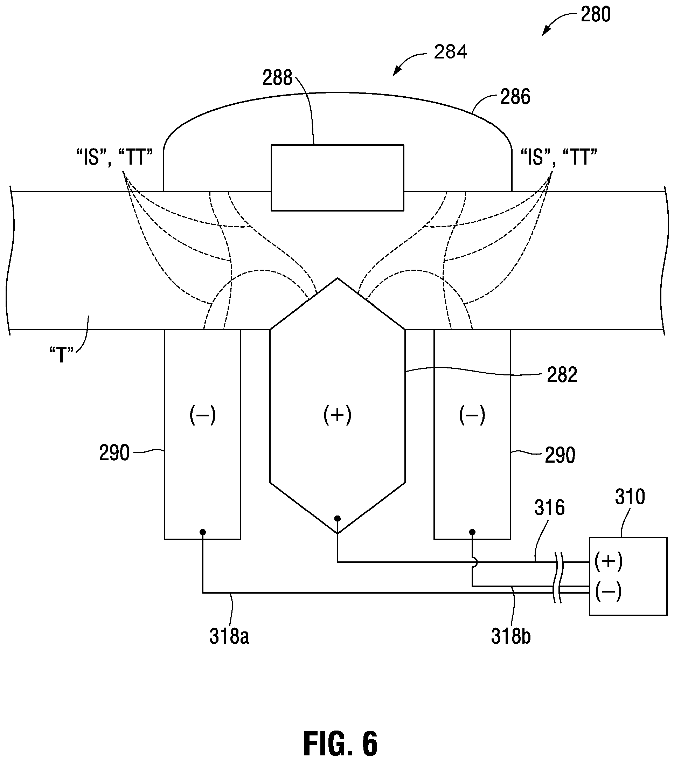

Turning to FIG. 6, in conjunction with FIGS. 1A-3, blade 282, structural body 286 of jaw 284, and/or panels 290 may additionally or alternatively be utilized to interrogate tissue "T," for example, to obtain feedback data relating thereto for use in feedback-based control of ultrasonic instrument 10. Further, blade 282, structural body 286 of jaw 284, and/or panels 290 may additionally or alternatively be utilized to create a bipolar RF circuit for conducting RF energy through tissue "T" to treat tissue "T" in conjunction with or separately from the application of ultrasonic energy thereto.

To enable tissue interrogation and/or tissue treatment, blade 282 may be electrically coupled to generator 310 via a first electrical path 316 which may, for example, extend between generator 310 and blade 282 by way of connectors 330, rotational contacts 334, horn 324, and waveguide 230 (see FIG. 2), or other suitable connections and/or components. Panels 290 are electrically coupled to generator 310 via connected or separate electrical paths 318a, 318b. Electrical paths 318a, 318b may include lead wires, contacts, and/or electrically-conductive components of ultrasonic instrument 10 that connect panels 290 with generator 310. For example, lead wires and/or portions of outer drive sleeve 210 or inner support sleeve 220 may electrically connect between panels 290 and associated contacts (not explicitly identified) disposed within body portion 112 of housing 110 of handle assembly 100 whereby the associated contacts are configured to mate with corresponding contacts (not explicitly identified) of TAG 300 upon engagement of TAG 300 with body portion 112 of housing 110 (see FIGS. 1A and 2). In configurations where the generator 1200 is remotely disposed and coupled to the ultrasonic surgical instrument 1010, e.g., by way of cable and plug assembly 1190, the lead wires 1199 extend through the cable and plug assembly 1190 o generator 1200 to connect panels 290 with generator 1200 (see FIG. 1B). Additionally or alternatively, structural body 286 of jaw 284 may be electrically coupled to generator 310 together with panels 290, e.g., via electrical paths 318a and/or 318b, or together with blade 282, e.g., via electrical path 316.

Continuing with reference to FIG. 6, in order to interrogate tissue "T," generator 310 is configured to transmit an interrogation signal "IS" to blade 282 via electrical path 316. Interrogation signal "IS" may be, for example, a Radio Frequency (RF) signal at a first potential, e.g., a positive potential (+). The interrogation signal "IS" may include one pulse or a plurality of pulses. Generator 310 also establishes a second, different potential at panels 290, e.g., a negative potential (-), such that the interrogation signal "IS" is transmitted from blade 282 through tissue "T" to panels 290 due to the potential difference therebetween. The signal is returned to generator 310 via electrical paths 318a, 318b, thus allowing generator 310 to evaluate the returned signal. In other embodiments, rather than panels 290 defining the same potential and blade 282 defining a different potential, panels 290 may define different potentials (with blade 282 not forming part of the interrogation circuit) to enable the interrogation signal "IS" to be transmitted from one panel 290, through tissue, and to the other panel 290. Further, as noted above, structural body 286 of jaw 284 may be electrically coupled to generator 310 and may define the same potential as either panels 290 or blade 282, e.g., to enable the interrogation signal "IS" to be transmitted between panels 290 and both of structural body 286 and blade 282, or between blade 282 and both of structural body 286 and panels 290. Alternatively, structural body 286 of jaw 284 may replace panels 290 or blade 282 in the circuit, e.g., to enable the interrogation signal "IS" to be transmitted between panels 290 and structural body 286 or between blade 282 and structural body 286.

Generator 310 is configured to evaluate the returned signal, e.g., the voltage, current, resistance, etc. thereof, and, based thereon, determine one or more parameters of tissue "T." For example, generator 310 may be configured to determine the impedance of tissue "T" which is indicative of whether tissue "T" is sufficiently sealed. As such, the interrogation signal "IS" may be utilized to determine whether tissue "T" is sufficiently sealed. If it is determined that tissue "T" is sufficiently sealed, generator 310 may be configured to produce an audible tone, visual indicator, haptic feedback, and/or other output to indicate to the surgeon that tissue "T" has been sufficiently sealed. Additionally or alternatively, determination of sufficiently sealed tissue may be utilized as part of a feedback loop to, for example, modify the ultrasonic drive signal output from generator 310, e.g., by increasing power, to facilitate cutting of the sealed tissue. Other tissue parameters may additionally or alternatively be utilized to determine whether tissue is sealed and/or for other purposes. For example, temperature measurement of one or both of panels 290 at one or more locations thereon, e.g., using thermocouples, may be taken and fed back to generator 310.

Continuing with reference to FIG. 6, as noted above, blade 282, structural body 286 of jaw 284, and/or panels 290 may additionally or alternatively be utilized to treat tissue with an additional form of energy (other than ultrasonic energy). More specifically, blade 282, structural body 286 of jaw 284, and/or panels 290 may create a bipolar RF circuit for conducting RF energy through tissue "T" to treat tissue "T" in conjunction with or separately from the application of ultrasonic energy thereto. That is, rather than or in addition to supplying an interrogation signal "IS" through tissue "T" as detailed above, a tissue-treating energy "TT" may be supplied. For example, a bipolar RF tissue-treating current "TT" may be conducted through tissue (using any of the electrical configurations detailed herein). That is, in order to treat tissue "T," generator 310 may be configured to energize blade 282 with RF energy at a first potential e.g., a positive potential (+), via electrical path 316, and to energize panels 290 with RF energy at a second, different potential e.g., e.g., a negative potential (-), via electrical paths 318a, 318b such that a potential gradient is established between blade 282 and panels 290 to enable the conduction of energy, e.g., a bipolar RF tissue-treating current "TT," through tissue disposed therebetween to treat the tissue disposed therebetween. Further, as noted above, structural body 286 of jaw 284 may be electrically coupled to generator 310 and may define the same potential as either panels 290 or blade 282, e.g., to enable the conduction of the bipolar RF tissue-treating current "TT" between panels 290 and both of structural body 286 and blade 282 to treat tissue therebetween, or between blade 282 and both of structural body 286 and panels 290 to treat tissue therebetween. Alternatively, structural body 286 of jaw 284 may replace panels 290 or blade 282 in the circuit, e.g., to enable the conduction of the bipolar RF tissue-treating current "TT" between panels 290 and structural body 286 to treat tissue therebetween or between blade 282 and structural body 286 to treat tissue therebetween.

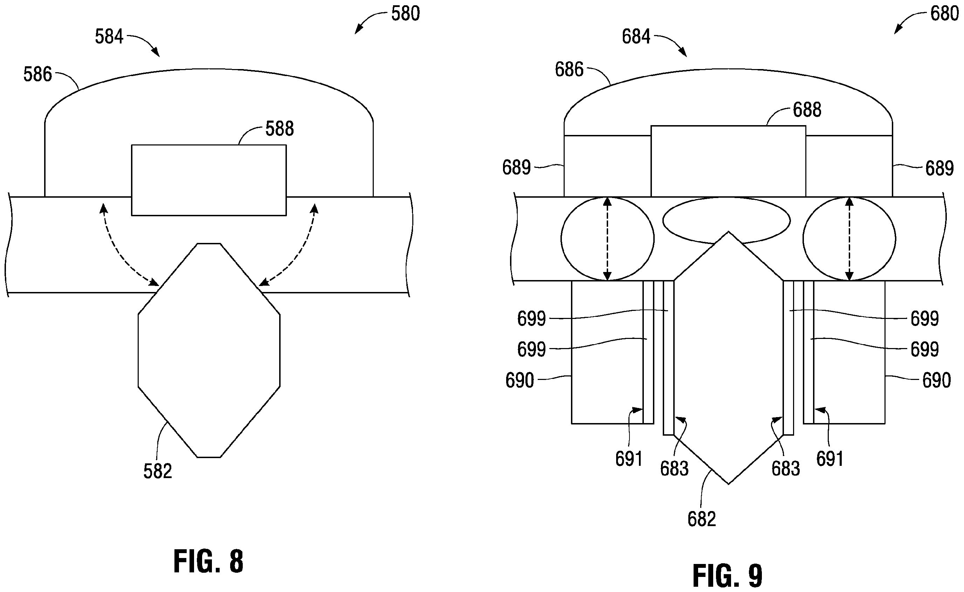

Although the exemplary interrogation signal "IS" and tissue-treating energy "TT" are detailed above utilizing RF energy, it is also contemplated that other forms of energy can be utilized such as, for example: microwave energy, thermal energy, optical energy (such as infrared energy), ultrasound energy, low frequency or direct current electrical energy, etc. Various configurations facilitating application of one or more of these energies for interrogating and/or treating tissue, in conjunction with ultrasonic tissue treatment, are detailed below with respect to FIGS. 8-19C.

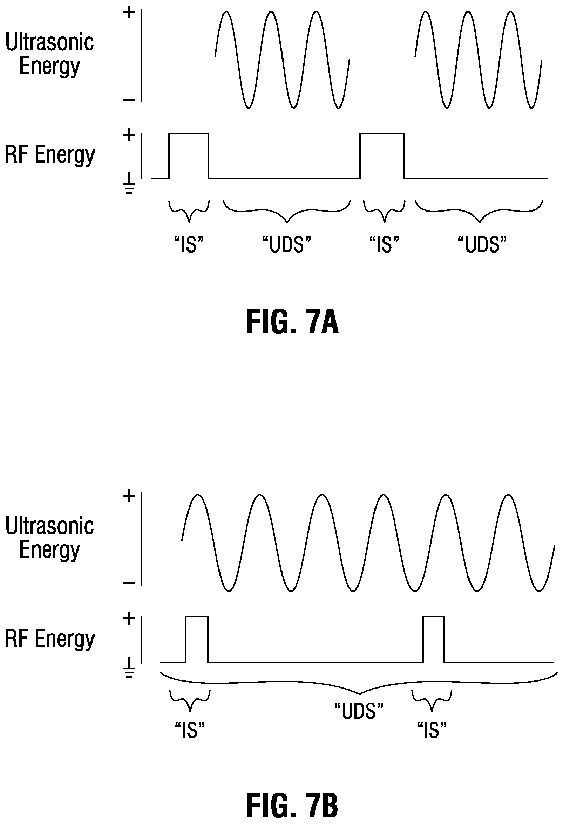

Turning to FIGS. 7A-7D, various energy-delivery configurations for supplying ultrasonic drive signals to tissue to treat tissue, interrogation signals to tissue to sense one or more parameters, and/or energy to tissue to treat tissue as a function of time are illustrated as graphical representations.

Referring to FIG. 7A, in conjunction with FIGS. 2, 5, and 6, in use, as noted above, generator 310 outputs a high voltage AC waveform ultrasonic drive signal "UDS" to drive piezoelectric stack 324 of ultrasonic transducer 320 such that ultrasonic energy is transmitted to ultrasonic horn 326, along waveguide 230, and to blade 282 to treat, e.g., seal and/or cut, tissue. Generator 310 may further be configured to stop output of the ultrasonic drive signal "UDS," output an interrogation signal "IS" (one or more pulses thereof) and, thereafter, resume output of the ultrasonic drive signal "UDS." The ultrasonic drive signal "UDS" may be stopped and the interrogation signal "IS" output periodically at fixed or variable intervals. For example, fewer stops and interrogation signals "IS" may be provided when activation is initiated and more stops and interrogation signals "IS" may be provided as activation time increases. In embodiments, rather than stopping output of the ultrasonic drive signal "UDS," the interrogation signals "IS" can be output concurrently with the ultrasonic drive signal "UDS," periodically, continuously, or in any other suitable manner.

The frequency with which the interrogation signals "IS" are output may additionally or alternatively depend upon the mode of operation and/or other factors. For example, interrogation signals "IS" may be provided more frequently, e.g., at shorter intervals, in the HIGH power mode of operation and less frequently, e.g., at longer intervals, in the LOW power mode of operation. As another example, the frequency with which the interrogation signals "IS" are output may depend upon the tissue parameter determined, e.g., the interrogation signals "IS" may be output more frequently as the impedance of tissue indicates that a sufficiently sealed condition is approaching. The number of pulses in each interrogation signal "IS" may also be fixed or variable similarly as with the frequency of the interrogation signal "IS" or in any other suitable manner.

With reference to FIG. 7B, as an alternative to stopping output of the ultrasonic drive signal "UDS" to output the interrogation signal "IS" and thereafter resuming output of the ultrasonic drive signal "UDS," the ultrasonic drive signal "UDS" may be continuously supplied while the interrogation signal "IS" is periodically output at fixed or variable intervals such that, while the interrogation signal "IS" is supplied, both the ultrasonic drive signal "UDS" and the interrogation signal "IS" are supplied simultaneously. The interrogation signal "IS" may otherwise be similar as detailed above.

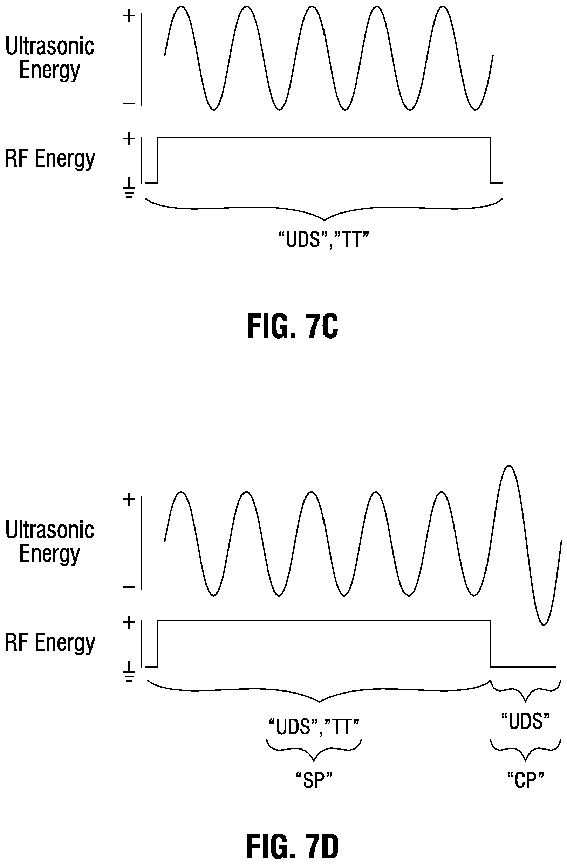

Referring to FIG. 7C, with respect to both supplying an ultrasonic drive signal "UDS," e.g., to drive piezoelectric stack 324 of ultrasonic transducer 320 such that ultrasonic energy is transmitted to ultrasonic horn 326, along waveguide 230, and to blade 282 to treat tissue (see FIGS. 3 and 5), and supplying other energy, e.g., conducting a bipolar RF tissue-treating current "TT" between blade 282, panels 290, and/or structural body 286 to treat tissue, the ultrasonic drive signal "UDS" and tissue treating energy "TT" may be supplied simultaneously or in at least partially overlapping temporal relation. Simultaneous ultrasonic and RF (or other additional energy) delivery to tissue may be utilized to seal tissue and/or achieve other tissue effects.

With reference to FIG. 7D, with respect to both supplying an ultrasonic drive signal "UDS" and supplying other energy, e.g., conducting a bipolar RF tissue-treating current "TT" between blade 282, panels 290, and/or structural body 286 to treat tissue, the ultrasonic drive signal "UDS" and tissue treating energy "TT" may, during a tissue sealing period "SP," be supplied simultaneously with the ultrasonic drive signal "UDS" at a first level. Once tissue is sealed, after a pre-determined time, upon receipt of a user-initiated signal, or based on other signals and/or feedback, a tissue cutting period "CP" is initiated wherein the tissue treating energy "TT" is stopped and ultrasonic drive signal "UDS" is increased from the first level to a second level, e.g., from a first amplitude to a second, greater amplitude, to increase the velocity of blade 282 (FIG. 5) to facilitate cutting the sealed tissue.

FIG. 8 illustrates another end effector assembly 580 in accordance with the present disclosure configured for use with any of the instruments or systems detailed herein. End effector assembly 580 is similar to end effector assembly 280 (FIGS. 3-5) except that end effector assembly 580 does not include panels.

With respect to the supply of ultrasonic energy using end effector assembly 580, ultrasonic energy is transmitted to blade 582 for treating tissue clamped between blade 582 and jaw liner 588 of jaw 584 or positioned near blade 582.

To supply other energy, e.g., RF energy, for interrogating tissue, the generator (not shown) is configured to transmit an interrogation signal to blade 582 via a first electrical path, e.g., an RF signal at a positive potential (+), and to establish a second, different potential structural body 586 of jaw 584, e.g., a negative potential (-), such that the interrogation signal is transmitted from blade 582 through tissue, to structural body 586 of jaw 584 due to the potential difference therebetween and returned to the generator via a second, different electrical path, thus allowing the generator to evaluate the returned signal.

With respect to the supply of other energy, e.g., RF energy, for treating tissue, the generator (not shown) is configured to energize blade 582 to a first potential via a first electrical path, e.g., RF energy at a positive potential (+), and to energize structural body 586 of jaw 584 to a second, different potential via a second, different electrical path, e.g., RF energy at a negative potential (-), to enable the conduction of energy, e.g., a bipolar RF tissue-treating current, through tissue disposed therebetween to treat the tissue disposed therebetween.

Referring to FIG. 9, another end effector assembly 680 is provided in accordance with the present disclosure and configured for use with any of the instruments or systems detailed herein. End effector assembly 680 is similar to end effector assembly 280 (FIGS. 3-5) and, thus, only the differences therebetween are described in detail below.

End effector assembly 680 includes a blade 682, a jaw 684 including a structural body 686, a jaw liner 688, and first and second electrically-conductive plates 689, and first and second panels 690 disposed on either side of blade 682. Structural body 686 supports first and second electrically-conductive plates 689 on either side of jaw liner 688. First and second electrically-conductive plates 689 may be electrically-isolated from structural body 686 or may be electrically coupled thereto (and, thus, to one another). First and second electrically-conductive plates 689 are configured to connect to the generator to enable energization thereof to a first potential, e.g., RF energy at a positive potential (+).

First and second panels 690, in addition or as an alternative to providing the abovedetailed function of panels 290 (FIGS. 3-5), function as electrodes that are configured to connect to the generator to enable energization thereof to a second, different potential, e.g., RF energy at a negative potential (-). In this manner, an RF tissue-treating current can be conducted between plates 689 and panels 690 and through tissue disposed therebetween to treat, e.g., seal, tissue. Plates 689 and panels 690 may additionally or alternatively be used to interrogate tissue to sense one or more parameters thereof, similarly as detailed above.

Continuing with reference to FIG. 9, with respect to RF tissue treatment or RF tissue interrogation, blade 682 may remain unenergized (with respect to RF energy; blade 682 may be energized with ultrasonic energy to ultrasonically treat tissue). To maintain isolation of blade 682, lateral surfaces 683 of blade 682 and/or inwardly-facing surfaces 691 of panels 690 may be coated with insulative coatings 699. The insulative coatings 699 may be PTFE or other suitable electrically-insulative coatings.

As an alternative to blade 682 being non-energized with RF energy, blade 682 may alternatively be energized to the same potential as either panels 690 or plates 689. The insulative coatings 699 isolate blade 682 from panels 690 as detailed above while jaw liner 688 isolates blade 682 from plates 689.

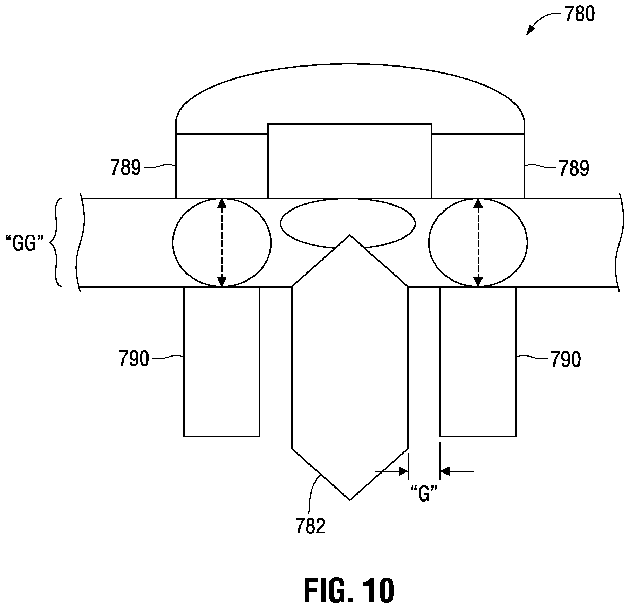

With reference to FIG. 10, yet another end effector assembly 780 is provided in accordance with the present disclosure and configured for use with any of the instruments or systems detailed herein. End effector assembly 780 is similar to end effector assembly 680 (FIG. 9) except that end effector assembly 780 does not include insulative coatings between blade 782 and panels 790. Rather, end effector assembly 780 is configured to maintain a gap distance "G" between blade 782 and each panel 790 at a suitable gap distance or within a suitable gap distance range to maintain electrical isolation therebetween. Likewise, end effector assembly 780 is configured to maintain a gap distance "GG," similar to or different from gap distance "G," between panels 790 and each electrically-conductive plate 789 at a suitable gap distance or within a suitable gap distance range to maintain electrical isolation therebetween. Either or both of the gap distances "G," "GG" may be suitable gap distances for sealing tissue, although other gap distances are also contemplated.

In use, first and second electrically-conductive plates 789 are configured to connect to the generator to enable energization thereof to a first potential, e.g., RF energy at a positive potential (+), while first and second panels 790 function as electrodes that are configured to connect to the generator to enable energization thereof to a second, different potential, e.g., RF energy at a negative potential (-), such that an RF tissue-treating current can be conducted between plates 789 and panels 790 and through tissue disposed therebetween to treat, e.g., seal, tissue. Plates 789 and panels 790 may additionally or alternatively be used to interrogate tissue to sense one or more parameters thereof, similarly as detailed above.

With respect to RF tissue treatment or RF tissue interrogation, blade 782 may remain unenergized (with respect to RF energy; blade 782 may be energized with ultrasonic energy to ultrasonically treat tissue). The gap distance(s) "G," "GG" maintain isolation of blade 782. As an alternative to blade 782 being non-energized with RF energy, blade 782 may alternatively be energized to the same potential as either panels 790 or plates 789 with gap distance(s) "G," "GG" isolating blade 782 therefrom.

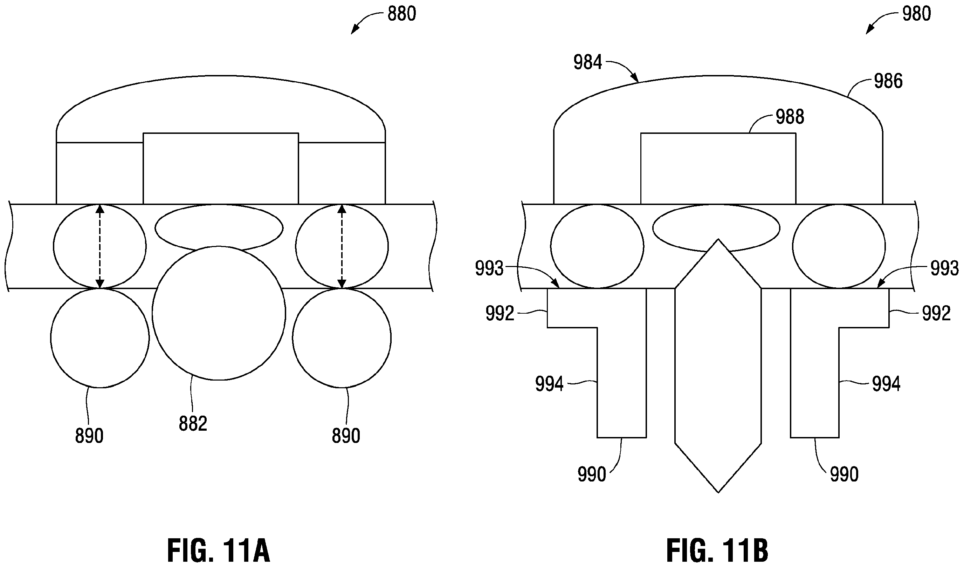

FIG. 11A illustrates still another end effector assembly 880 in accordance with the present disclosure configured for use with any of the instruments or systems detailed herein. End effector assembly 880 is similar to end effector assembly 780 (FIG. 10) except that blade 882 defines a cylindrical configuration and/or instead of panels, cylindrical rods 890 are provided. In some configurations, insulative coatings are provided between blade 882 and rods 890, similarly as detailed above with respect to end effector assembly 680 (FIG. 9).

FIG. 11B illustrates still yet another end effector assembly 980 in accordance with the present disclosure and configured for use with any of the instruments or systems detailed herein. End effector assembly 980 is similar to end effector assembly 780 (FIG. 10) except that panels 990 define shelves 992 extending perpendicularly and outwardly from the vertical bodies 994 thereof. As a result, shelves 992 define surfaces 993 that extend in generally parallel orientation (e.g., within about 15 degrees) of the opposing surface of structural body 986 of jaw 984 in the closed position of jaw 984. Instead of structural body 986 defining the opposing surfaces, jaw 984 may include electrically-conductive plates similarly as detailed above.

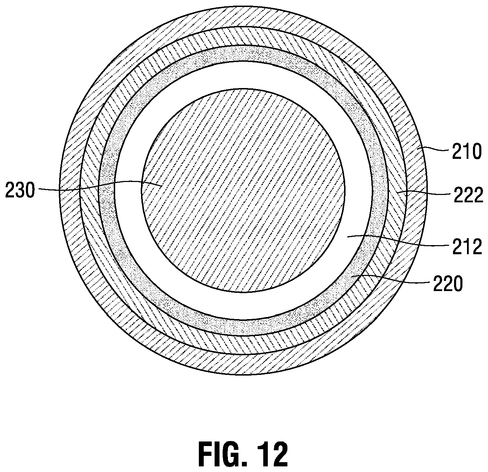

Turning to FIG. 12, in conjunction with FIGS. 2-5, in embodiments, the electrical paths connecting the generator with panels 290 and the generator with jaw 284 (the structural body and/or electrically-conductive plates thereof) may be made at least partially by lead wires. Alternatively, structural components may be utilized. For example, outer tube 210 may define at least a portion of the electrical path connecting the generator with panels 290 while inner tube 220 defines at least a portion of the electrical path connecting the generator with jaw 284. In such configurations, first and second insulators 212, 222 are provided to electrically isolate waveguide 230, inner tube 220, and outer tube 210 from one another. Insulators 212, 222 may be configured as sheaths, spaced-apart rings, or in any other suitable manner so as to maintain electrical isolation. Waveguide 230, insulators 212, 222, inner tube 220, and outer tube 210 may be concentrically disposed relative to one another.

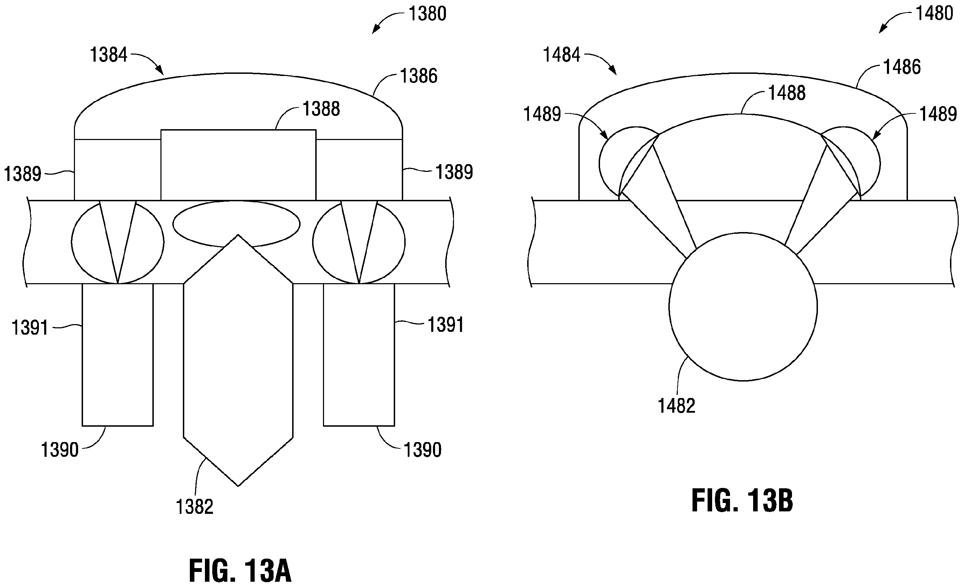

Referring to FIG. 13A, another end effector assembly 1380 is provided in accordance with the present disclosure and configured for use with any of the instruments or systems detailed herein. End effector assembly 1380 includes a blade 1382, a jaw 1384 including a structural body 1386, a jaw liner 1388, and first and second optical elements 1389, and first and second panels 1390 disposed on either side of blade 1382 and configured as optical elements 1391. Structural body 1386 supports first and second optical elements 1389 on either side of jaw liner 1388. First and second optical elements 1389 may be similar or different and may be configured as optical emitters, optical absorbers, or optical reflectors. Likewise, the optical elements 1391 of panels 1390 are similar or different and may be configured as optical emitters, optical absorbers, or optical reflectors such that each pair of opposing optical elements 1389, 1391 includes one optical emitter and one optical absorber or optical reflector.

The optical elements 1389, 1391 configured as optical emitters are configured to emit light energy and direct the light energy through tissue disposed between optical elements 1389, 1391. The optical elements 1389, 1391 configured as optical absorbers or optical reflectors are configured to absorb light energy that has passed through tissue or reflect the light energy that has passed through tissue back into tissue, respectively. This light energy passing through tissue may be utilized to treat, e.g., seal, tissue, and/or to interrogate tissue. The light energy may include infrared light, ultraviolet light, visible light, laser light, or any other suitable light or combinations thereof.

FIG. 13B illustrates another end effector assembly 1480 provided in accordance with the present disclosure and configured for use with any of the instruments or systems detailed herein. End effector assembly 1480 includes a blade 1482 and a jaw 1484 including a structural body 1486, a jaw liner 1488 that is transparent to the optical wavelengths used (made from, for example, a PTFE which is substantially transparent at ultraviolet [UV] wavelengths (although other materials and corresponding optical transparency wavelengths are also contemplated), and first and second optical elements 1489 mounted on or within structural body 1486. First and second optical elements 1486, more specifically, are oriented to emit light energy through transparent jaw liner 1488 and through tissue disposed between jaw 1484 and blade 1482. Blade 1482 may act as an optical absorber or reflector, in embodiments, and may include coatings or other suitable features to enhance the absorption or reflection thereof.

First and second optical elements 1489 may be oriented to direct light energy towards blade 1482 in a generally perpendicular direction relative to the outer surface of blade 1482. More specifically, in some aspects, blade 1482 may define a cylindrical configuration with first and second optical elements 1489 oriented to direct light energy in directions that intersect a center of blade 1482, as shown in FIG. 13B.

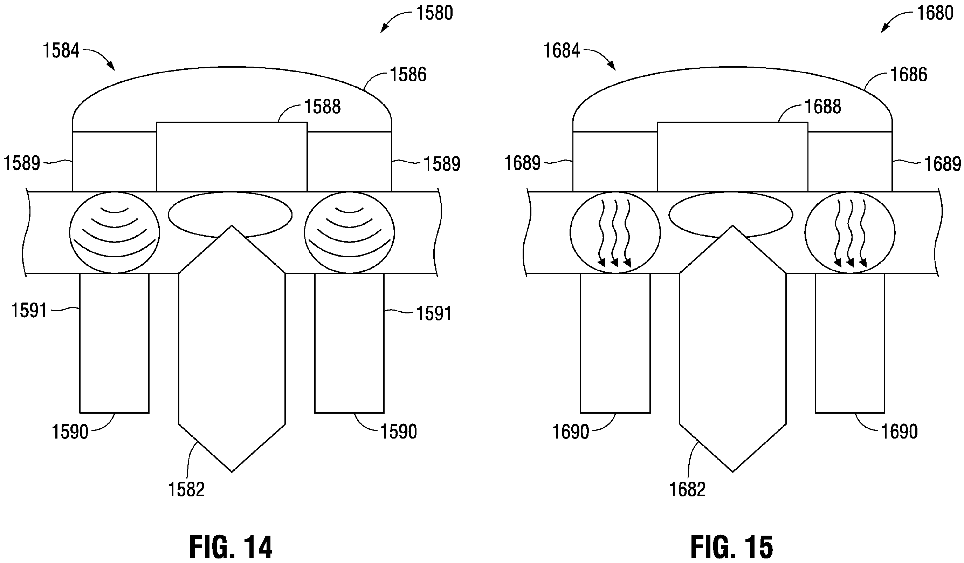

Referring to FIG. 14, another end effector assembly 1580 is provided in accordance with the present disclosure and configured for use with any of the instruments or systems detailed herein. End effector assembly 1580 includes a blade 1582, a jaw 1584 including a structural body 1586, a jaw liner 1588, and first and second ultrasound transducers 1589, and first and second panels 1590 disposed on either side of blade 1582 and configured as ultrasound absorbers or reflectors 1591. Structural body 1586 supports the ultrasound transducers 1589 on either side of jaw liner 1588. First and second ultrasound transducers 1589 are configured to produce and emit ultrasound waves towards panels 1590 and through tissue disposed between jaw 1584 and panels 1590, e.g., to treat tissue, to image tissue, and/or to interrogate tissue. The ultrasound absorbers or reflectors 1591 are configured to absorb or reflect the ultrasound waves that have passed through tissue. Alternatively, this configuration may be reversed on either or both sides, e.g., where one or both ultrasound transducer(s) 1589 are disposed on the corresponding panel(s) 1590 and where the absorber(s) or reflector(s) 1591 are disposed on the corresponding side(s) of structural body 1586.

Turning to FIG. 15, still another end effector assembly 1680 is provided in accordance with the present disclosure and configured for use with any of the instruments or systems detailed herein. End effector assembly 1680 includes a blade 1682, a jaw 1684 including a structural body 1686, a jaw liner 1688, and first and second heating elements 1689, and first and second panels 1690 disposed on either side of blade 1682. Panels 1690 may, in some configurations, also include heating elements. First and second heating elements 1689 may include heating coils configured to produce thermal energy in response to energization of the heating coils. This thermal energy (and the thermal energy from panels 1690, when configured to include heating elements), is transmitted to tissue disposed between jaw 1684 and panels 1690, e.g., to treat tissue.

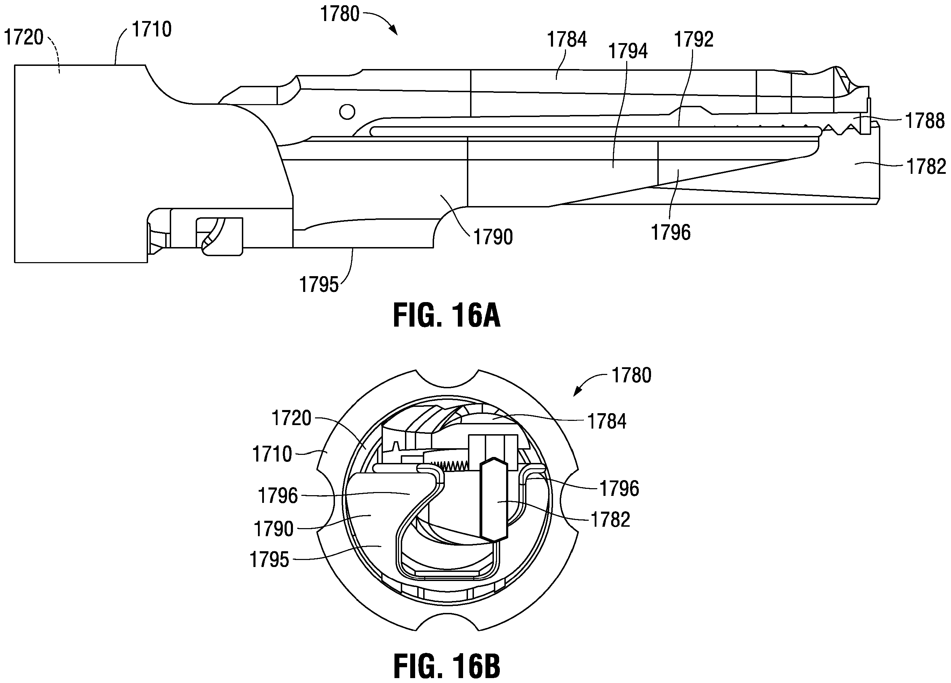

FIGS. 16A and 16B illustrate another end effector assembly 1780 similar to end effector assembly 280 (FIGS. 3-5) that may include any of the features thereof and/or of any other end effector assembly detailed herein. End effector assembly 1780 differs from end effector assembly 280 (FIGS. 3-5) in the configuration of the panels and, thus, only those differences are detailed hereinbelow.

End effector assembly 1780 includes a panel member 1790 having a base 1795 surrounding, on the bottom and lateral sides, a portion of blade 1782, and first and second panels 1796 extending distally from base 1795 on either lateral side of blade 1782.

Base 1795 defines a U-shaped configuration and extends, in fixed or movable relation, distally from outer and/or inner sleeves 1710, 1720. Base 1795, more specifically, extends less than about 50% of the operative length of blade 1782 (defines as the portion of blade 1782 that opposes the jaw liner 1788 of jaw 1784 in the closed position), less than about 40% of the operative length of blade 1782, or less than about 30% of the operative length of blade 1782. As a result, the bottom portion of blade 1782 is exposed along the distal 50%, 60%, or 70% of the operative length thereof.

Panels 1796 extend distally from base 1795 on either side of blade 1782. Panels 1796 may include shelves 1792 extending perpendicularly and outwardly from upper ends of the vertical bodies 1794 thereof. Panels 1796 may further include tapers, from the lower ends thereof, whereby the height of panels 1796 decreasingly tapers from the proximal end thereof towards the distal end thereof. As a result, the bottom portion and lateral sides of blade 1782 are increasing exposed in a proximal-to-distal direction. This configuration facilitates use of the distal, bottom portion of blade 1782, e.g., for enterotomies, backscoring, and/or or other surgical tasks, while still providing the functionality of the panels as detailed with respect to any of the above configurations.

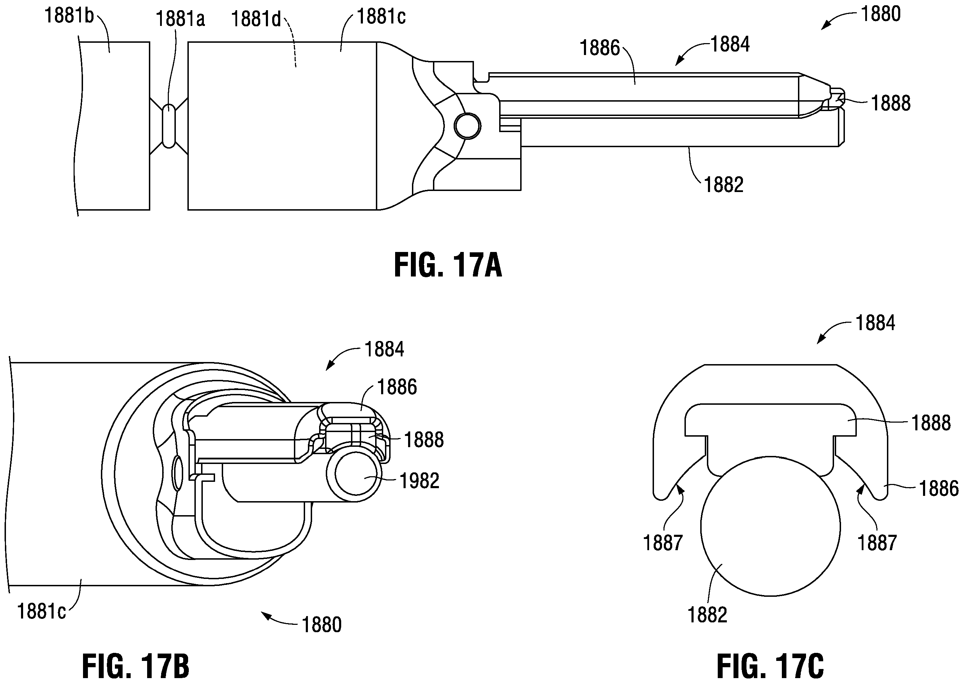

Referring generally to FIGS. 17A-18C (and also applicable to FIGS. 6-16), as opposed to a handle assembly 100 (FIG. 1A) for handheld, manual manipulation and operation, the various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

Referring in particular to FIGS. 17A-17C, an end effector assembly 1880 for use with a robotic surgical system is shown, although end effector assembly 1880 may alternatively be used in a handheld instrument and/or any of the other end effector assemblies detailed herein may be utilized with a robotic surgical system. End effector assembly 1880 is positioned distally of one of more articulation joints 1881a of a robotic shaft assembly 1881b and includes a proximal housing 1881c supporting an ultrasonic transducer 1881d therein or thereon. A waveguide (not explicitly shown) extends distally form the ultrasonic transducer 1881d and includes blade 1882 defined at a distal end thereof. A jaw 1884 is pivotably mounted to the housing 1881c to enable pivoting of the jaw 1884 relative to the blade 1882 between open and clamping positions for clamping tissue between the jaw 1884 and blade 1882. Blade 1882 defines a cylindrical configuration, although other configurations are also contemplated. In some configurations, jaw 1884 is capable of rotating about blade 1882.

Jaw 1884 includes a structural body 1886 and a jaw liner 1888. Structural body 1886 itself or electrically-conductive plates (not shown) mounted thereon may be configured to connect to the generator to serve as one electrode (or portion) of an RF (or other energy-based) circuit. Blade 1882 is configured to connect to the generator to serve as the other electrode (or portion) of the RF (or other energy-based) circuit. The opposed surfaces 1887 of structural body 1886 (or the opposed surfaces of electrically-conductive plates, if so provided) are radiused to complement the cylindrical configuration of blade 1882 such that the gap distance between opposed surfaces 1887 and blade 1882 with tissue grasped therebetween is substantially (e.g., within about 15%) uniform at any point therealong. RF signals may be communicated between structural body 1886 and blade 1882 to interrogate and/or treat tissue similarly as detailed above. Other suitable energy configurations are also contemplated such as those detailed above.

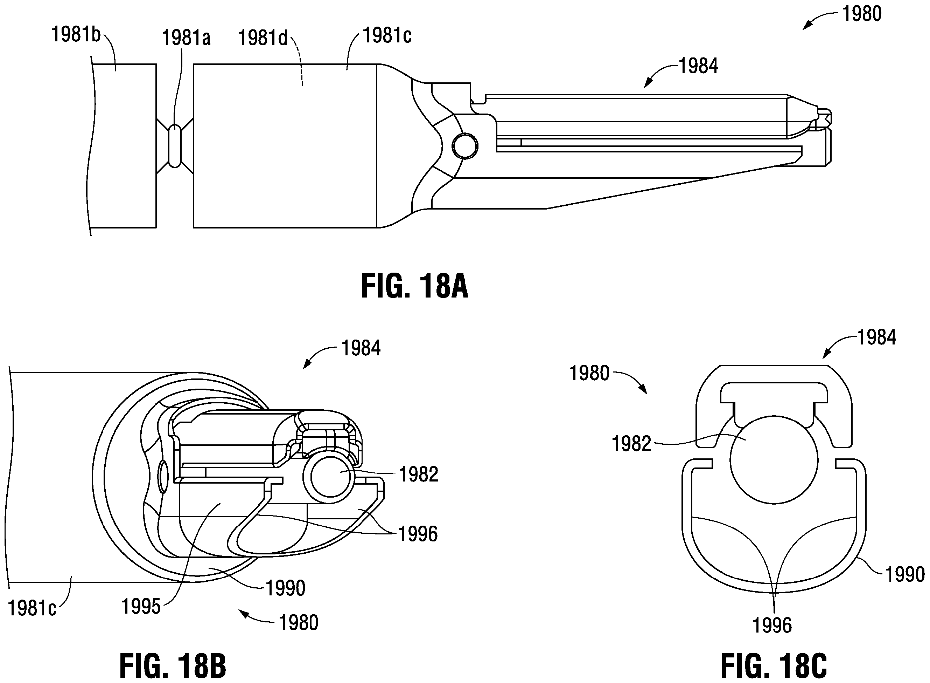

With reference to FIGS. 18A-18C, another end effector assembly 1980 for use with a robotic surgical system is shown, although end effector assembly 1880 may alternatively be used in a handheld instrument. End effector assembly 1980 is similar to end effector assembly 1880 (FIGS. 17A-17C) and may include any of the features of end effector assembly 1880 (FIGS. 17A-17C) or any other end effector assembly detailed herein.

End effector assembly 1980 is positioned distally of one of more articulation joints 1981a of a robotic shaft assembly 1981b and includes a proximal housing 1981c supporting an ultrasonic transducer 1981d therein. A waveguide (not explicitly shown) extends distally form the ultrasonic transducer 1981d and includes a blade 1982 defined at a distal end thereof. A jaw 1984 is pivotably mounted to the housing 1981c to enable pivoting of the jaw 1984 relative to the blade 1982 between open and clamping positions for clamping tissue between the jaw 1984 and blade 1982.

End effector assembly 1980 differs from end effector assembly 1880 (FIGS. 17A-17C) in that end effector assembly 1980 further includes a panel member 1990 having a base 1995 surrounding, on the bottom and lateral sides, a portion of blade 1982, and first and second tapered panels 1996 extending distally from base 1995 on either lateral side of blade 1982. Panel member 1990 may be similar to and include any of the features of panel member 1790 of end effector assembly 1780 (see FIGS. 16A and 16B).

While several embodiments of the disclosure have been detailed above and are shown in the drawings, it is not intended that the disclosure be limited thereto, as it is intended that the disclosure be as broad in scope as the appended claims will allow and that the specification be read likewise. Therefore, the above description and accompanying drawings should not be construed as limiting, but merely as exemplifications of particular embodiments. Those skilled in the art will envision other modifications within the scope of the claims appended hereto

## Claims

1. A surgical end effector assembly (10), comprising:
an ultrasonic blade (282) adapted to connect to an ultrasonic transducer configured to transmit ultrasonic energy to the ultrasonic blade; **characterized in that**, the assembly further comprises
first and second panels (290) disposed on opposite sides of the ultrasonic blade in laterally-spaced relation relative thereto; and
a jaw (284) pivotable relative to the ultrasonic blade and the first and second panels between open and clamping positions,
wherein at least two of the ultrasonic blade, the first and second panels, or the jaw are energizable to different electrical potentials for transmitting an electric signal therebetween and through tissue for at least one of interrogating tissue or treating tissue.

2. The surgical end effector assembly according to claim 1, wherein the ultrasonic blade is configured to be energized to a first potential and the first and second panels are configured to be energized to a second, different potential for transmitting the electric signal therebetween and through tissue.

3. The surgical end effector assembly according to claim 1, wherein the jaw is configured to be energized to a first potential and the first and second panels are configured to be energized to a second, different potential for transmitting the electric signal therebetween and through tissue.

4. The surgical end effector assembly according to claim 3, wherein the jaw (284) includes a structural body (286) defining first and second surfaces (299a, 299b), and a jaw liner (288) disposed between the first and second surfaces, the first and second surfaces energizable for transmitting the electric signal.

5. The surgical end effector assembly according to claim 3 or 4, wherein the jaw includes a structural body supporting first and second electrically-conductive plates, and a jaw liner disposed between the first and second electrically-conductive plates, the first and second electrically-conductive plates energizable for transmitting the electric signal.

6. The surgical end effector assembly according to any preceding claim, further comprising an insulative coating disposed on at least one of outer lateral sides of the ultrasonic blade or inner lateral sides of the first and second panels.

7. The surgical end effector assembly according to any preceding claim, wherein a gap distance is maintained between outer lateral sides of the ultrasonic blade and inner lateral sides of the first and second panels.

8. The surgical end effector assembly according to any preceding claim, wherein each of the first and second panels includes a vertical body and a shelf extending laterally from the vertical body, the shelf defining an opposing surface positioned to oppose the jaw in the clamping position of the jaw.

9. The surgical end effector assembly according to any preceding claim, wherein each of the first and second panels tapers in height in a proximal-to-distal direction from lower portions thereof to increasingly expose a bottom portion of the blade in a proximal-to-distal direction.

10. The surgical end effector assembly according to any preceding claim, wherein the electric signal is a Radio Frequency (RF) signal.

11. The surgical end effector assembly according to any one of claims 1 to 9, wherein the electric signal is a Direct Current (DC) signal.

12. A surgical end effector assembly (10), comprising:
an ultrasonic blade (282) adapted to connect to an ultrasonic transducer (320) configured to transmit ultrasonic energy to the ultrasonic blade; **characterized in that**, the assembly further comprises
first and second panels (290) disposed on opposite sides of the ultrasonic blade in laterally-spaced relation relative thereto; and
a jaw (284) pivotable relative to the ultrasonic blade and the first and second panels between open and clamping positions, the jaw including a structural body (286), a jaw liner (288) supported by the structural body and positioned to oppose the ultrasonic blade in the clamping position of the jaw, and first and second energy elements supported by the structural body on either side of the jaw liner and positioned to oppose the first and second panels, respectively, in the clamping position of the jaw;
wherein the first and second energy elements are configured to facilitate delivery of another energy to tissue disposed between the first and second energy elements and the first and second panels, respectively.

13. The surgical end effector assembly according to claim 12, wherein the first and second energy elements are optical emitters configured to emit optical energy through tissue disposed between the first and second energy elements and the first and second panels, respectively, preferably wherein the first and second panels are configured as optical absorbers or optical reflectors.

14. The surgical end effector assembly according to claim 12 or 13, wherein the first and second energy elements are ultrasound transducers configured to emit ultrasound wave energy through tissue disposed between the first and second energy elements and the first and second panels, respectively, preferably wherein the first and second panels are configured as ultrasound absorbers or ultrasound reflectors.

15. The surgical end effector assembly according to claim 12, 13 or 14 wherein the first and second energy elements are thermal heaters configured to conduct thermal energy to tissue disposed between the first and second energy elements and the first and second panels, respectively.

## Patentansprüche

1. Chirurgische Greiforgananordnung (10), umfassend:
eine Ultraschallklinge (282), die dazu ausgelegt ist, mit einem Ultraschallwandler verbunden zu werden, der zum Senden von Ultraschallenergie zur Ultraschallklinge ausgebildet ist;
**dadurch gekennzeichnet, dass** die Anordnung ferner umfasst:
eine erste und eine zweite Platte (290), die auf gegenüberliegenden Seiten der Ultraschallklinge in lateral beabstandeter Beziehung in Bezug darauf angeordnet sind; und
eine Backe (284), die in Bezug auf die Ultraschallklinge und die erste und die zweite Platte zwischen einer offenen und einer Klemmposition drehbar ist,
wobei mindestens zwei der Ultraschallklinge, der ersten und der zweiten Platte oder der Backe zum Senden eines elektrischen Signals dazwischen und durch Gewebe für mindestens eines von Abfragegewebe oder Behandlungsgewebe auf verschiedene elektrische Potenziale erregbar sind.

2. Chirurgische Greiforgananordnung nach Anspruch 1, wobei die Ultraschallklinge dazu ausgebildet ist, auf ein erstes Potenzial erregt zu werden, und die erste und die zweite Platte dazu ausgebildet sind, auf ein zweites, verschiedenes Potenzial erregt zu werden, um das elektrische Signal dazwischen und durch Gewebe zu senden.

3. Chirurgische Greiforgananordnung nach Anspruch 1, wobei die Backe dazu ausgebildet ist, auf ein erstes Potenzial erregt zu werden, und die erste und die zweite Platte dazu ausgebildet sind, auf ein zweites, verschiedenes Potenzial erregt zu werden, um das elektrische Signal dazwischen und durch Gewebe zu senden.

4. Chirurgische Greiforgananordnung nach Anspruch 3, wobei die Backe (284) einen Strukturkörper (286), der eine erste Fläche und eine zweite Fläche (299a, 299b) definiert, und eine Backenauskleidung (288) umfasst, die zwischen der ersten und der zweiten Fläche angeordnet ist, wobei die erste und die zweite Fläche zum Senden des elektrischen Signals erregbar sind.

5. Chirurgische Greiforgananordnung nach Anspruch oder 2 oder 3, wobei die Backe einen Strukturkörper (286), der ein erstes und ein zweites elektrisch leitendes Plattenelement trägt, und eine Backenauskleidung umfasst, die zwischen dem ersten und dem zweiten elektrisch leitenden Plattenelement angeordnet ist, wobei das erste und das zweite elektrisch leitende Plattenelement zum Senden des elektrischen Signals erregbar sind.

6. Chirurgische Greiforgananordnung nach einem der vorhergehenden Ansprüche, ferner umfassend eine Isolierbeschichtung, die auf mindestens einer von lateralen Außenseiten der Ultraschallklinge oder lateralen Innenseiten der ersten und der zweiten Platte angeordnet ist.

7. Chirurgische Greiforgananordnung nach einem der vorhergehenden Ansprüche, wobei ein Abstandsspalt zwischen lateralen Außenseiten der Ultraschallklinge und lateralen Innenseiten der ersten und der zweiten Platte aufrechterhalten wird.

8. Chirurgische Greiforgananordnung nach einem der vorhergehenden Ansprüche, wobei jede der ersten und der zweiten Platte einen vertikalen Körper und einen Vorsprung umfasst, der sich lateral vom vertikalen Körper erstreckt, wobei der Vorsprung eine gegenüberliegende Fläche definiert, die so positioniert ist, dass sie der Backe in der Klemmposition der Backe gegenüberliegt.

9. Chirurgische Greiforgananordnung nach einem der vorhergehenden Ansprüche, wobei jede der ersten und der zweiten Platte in der Höhe in einer proximal-distalen Richtung von ihrem unteren Abschnitt konisch zuläuft, um einen unteren Abschnitt der Klinge in einer proximal-distalen Richtung zunehmend freizulegen.

10. Chirurgische Greiforgananordnung nach einem der vorhergehenden Ansprüche, wobei das elektrische Signal ein Radiofrequenzsignal (RF-Signal) ist.

11. Chirurgische Greiforgananordnung nach einem der Ansprüche 1 bis 9, wobei das elektrische Signal ein Gleichstromsignal (DC-Signal) ist.

12. Chirurgische Greiforgananordnung (10), umfassend:
eine Ultraschallklinge (282), die dazu ausgelegt ist, mit einem Ultraschallwandler (320) verbunden zu werden, der zum Senden von Ultraschallenergie zur Ultraschallklinge ausgebildet ist;
**dadurch gekennzeichnet, dass** die Anordnung ferner umfasst:
eine erste und eine zweite Platte (290), die auf gegenüberliegenden Seiten der Ultraschallklinge in lateral beabstandeter Beziehung in Bezug darauf angeordnet sind; und
eine Backe (284), die in Bezug auf die Ultraschallklinge und die erste und die zweite Platte zwischen einer offenen und einer Klemmposition drehbar ist, wobei die Backe einen Strukturkörper (286), eine Backenauskleidung (288), die vom Strukturkörper getragen wird und so positioniert ist, dass sie der Ultraschallklinge in der Klemmposition der Backe gegenüberliegt, und ein erstes und ein zweites Energieelement umfasst, die vom Strukturkörper auf jeder Seite der Backenauskleidung getragen werden und so positioniert sind, dass sie der ersten bzw. der zweiten Platte in der Klemmposition der Backe gegenüberliegen;
wobei das erste und das zweite Energieelement dazu ausgebildet sind, Zufuhr einer anderen Energie zu Gewebe zu ermöglichen, das zwischen dem ersten und dem zweiten Energieelement bzw. der ersten und der zweiten Platte angeordnet ist.

13. Chirurgische Greiforgananordnung nach Anspruch 12, wobei das erste und das zweite Energieelement optische Emitter sind, die zum Emittieren optischer Energie durch Gewebe ausgebildet sind, das zwischen dem ersten und dem zweiten Energieelement bzw. der ersten und der zweiten Platte angeordnet ist, wobei die erste und die zweite Platte vorzugsweise als optische Absorber oder optische Reflektoren ausgebildet sind.

14. Chirurgische Greiforgananordnung nach Anspruch 12 oder 13, wobei das erste und das zweite Energieelement Ultraschallwandler sind, die zum Emittieren von Ultraschallwellenenergie durch Gewebe ausgebildet sind, das zwischen dem ersten und dem zweiten Energieelement bzw. der ersten und der zweiten Platte angeordnet ist, wobei die erste und die zweite Platte vorzugsweise als Ultraschallabsorber oder Ultraschallreflektoren ausgebildet sind.

15. Chirurgische Greiforgananordnung nach Anspruch 12, 13 oder 14, wobei das erste und das zweite Energieelement Thermoheizelemente sind, die dazu ausgelegt sind, Wärmeenergie zu Gewebe zu leiten, das zwischen dem ersten und dem zweiten Energieelement bzw. der ersten und der zweiten Platte angeordnet ist.

## Revendications

1. Ensemble effecteur terminal chirurgical (10), comprenant :
une lame ultrasonore (282) adaptée pour se connecter à un transducteur ultrasonore configuré pour transmettre de l'énergie ultrasonore à la lame ultrasonore ;
**caractérisé en ce que**, l'ensemble comprend en outre :
un premier et un second panneaux (290) disposés sur des côtés opposés de la lame ultrasonore en relation d'espacement latéral par rapport à celle-ci ; et
une mâchoire (284) pouvant pivoter par rapport à la lame ultrasonore et aux premier et second panneaux entre des positions d'ouverture et de serrage,
au moins deux de la lame ultrasonore, des premier et second panneaux ou de la mâchoire pouvant être excités à des potentiels électriques différents pour transmettre un signal électrique entre eux et à travers le tissu pour au moins l'un d'un tissu d'interrogation ou d'un tissu de traitement.

2. Ensemble effecteur terminal chirurgical selon la revendication 1, la lame ultrasonore étant configurée pour être excitée à un premier potentiel et les premier et second panneaux étant configurés pour être excités à un second potentiel différent pour transmettre le signal électrique entre eux et à travers le tissu.

3. Ensemble effecteur terminal chirurgical selon la revendication 1, la mâchoire étant configurée pour être excitée à un premier potentiel et les premier et second panneaux étant configurés pour être excités à un second potentiel différent pour transmettre le signal électrique entre eux et à travers le tissu.

4. Ensemble effecteur terminal chirurgical selon la revendication 3, la mâchoire (284) comprenant un corps structurel (286) définissant des première et seconde surfaces (299a, 299b), et un revêtement de mâchoire (288) disposé entre les première et seconde surfaces, les première et seconde surfaces pouvant être excitées pour transmettre le signal électrique.

5. Ensemble effecteur terminal chirurgical selon la revendication 3 ou 4, la mâchoire comprenant un corps structurel supportant des première et seconde plaques électriquement conductrices, et un revêtement de mâchoire disposé entre les première et seconde plaques électriquement conductrices, les première et seconde plaques électriquement conductrices pouvant être excitées pour transmettre le signal électrique.

6. Ensemble effecteur terminal chirurgical selon n'importe quelle revendication précédente, comprenant en outre un revêtement isolant disposé sur au moins l'un des côtés latéraux extérieurs de la lame ultrasonore ou des côtés latéraux intérieurs des premier et second panneaux.

7. Ensemble effecteur terminal chirurgical selon n'importe quelle revendication précédente, une distance d'espacement étant maintenue entre des côtés latéraux extérieurs de la lame ultrasonore et des côtés latéraux intérieurs des premier et second panneaux.

8. Ensemble effecteur terminal chirurgical selon n'importe quelle revendication précédente, chacun des premier et second panneaux comprenant un corps vertical et une tablette s'étendant latéralement depuis le corps vertical, la tablette définissant une surface opposée positionnée pour s'opposer à la mâchoire dans la position de serrage de la mâchoire.

9. Ensemble effecteur terminal chirurgical selon n'importe quelle revendication précédente, chacun des premier et second panneaux s'effilant en hauteur dans une direction proximale à distale à partir de ses parties inférieures pour exposer de plus en plus une partie inférieure de la lame dans une direction proximale à distale.

10. Ensemble effecteur terminal chirurgical selon n'importe quelle revendication précédente, le signal électrique étant un signal de radiofréquence (RF).

11. Ensemble effecteur terminal chirurgical selon l'une quelconque des revendications 1 à 9, le signal électrique étant un signal de courant continu (CC).

12. Ensemble effecteur terminal chirurgical (10), comprenant :
une lame ultrasonore (282) adaptée pour se connecter à un transducteur ultrasonore (320) configuré pour transmettre une énergie ultrasonore à la lame ultrasonore ;
**caractérisé en ce que**, l'ensemble comprend en outre :
des premier et second panneaux (290) disposés sur des côtés opposés de la lame ultrasonore en relation d'espacement latéral par rapport à celle-ci ; et
une mâchoire (284) pouvant pivoter par rapport à la lame ultrasonore et aux premier et second panneaux entre des positions d'ouverture et de serrage, la mâchoire comprenant un corps structurel (286), un revêtement de mâchoire (288) supporté par le corps structurel et positionné pour s'opposer à la lame ultrasonore dans la position de serrage de la mâchoire, et des premier et second éléments d'énergie supportés par le corps structurel de chaque côté du revêtement de mâchoire et positionnés pour s'opposer aux premier et second panneaux, respectivement, dans la position de serrage de la mâchoire ;
les premier et second éléments d'énergie étant configurés pour faciliter la distribution d'une autre énergie au tissu disposé entre les premier et second éléments d'énergie et les premier et second panneaux, respectivement.

13. Ensemble effecteur terminal chirurgical selon la revendication 12, les premier et second éléments d'énergie étant des émetteurs optiques configurés pour émettre une énergie optique à travers le tissu disposé entre les premier et second éléments d'énergie et les premier et second panneaux, respectivement, de préférence, les premier et second panneaux étant configurés comme des absorbeurs optiques ou des réflecteurs optiques.

14. Ensemble effecteur terminal chirurgical selon la revendication 12 ou 13, les premier et second éléments d'énergie étant des transducteurs ultrasonores configurés pour émettre une énergie d'ondes ultrasonores à travers le tissu disposé entre les premier et second éléments d'énergie et les premier et second panneaux, respectivement, de préférence les premier et second panneaux étant configurés comme des absorbeurs d'ultrasons ou des réflecteurs d'ultrasons.

15. Ensemble effecteur terminal chirurgical selon la revendication 12, 13 ou 14, les premier et second éléments d'énergie étant des réchauffeurs thermiques configurés pour conduire l'énergie thermique au tissu disposé entre les premier et second éléments d'énergie et les premier et second panneaux, respectivement.
